# EUROPEAN PATENT SPECIFICATION

(11) **EP 1 641 457 B1**
(45) Date of publication and mention of the grant of the patent: **05.08.2009**
(21) Application number: 04741933.8
(22) Date of filing: 30.06.2004
(51) Int. Cl.: A61K 31/4745, A61P 25/00, A61P 35/00, C07D 471/06

(54) **PYRROLO-DIHYDROISOQUINOLINE DERIVATIVES AS PDE10 INHIBITORS**
PYRROLO-DIHYDROISOCHINOLIN-DERIVATE ALS PDE10-INHIBITOREN
DERIVES DE PYRROLO-DIHYDROISOQUINOLINES EN TANT QU' INHIBITEURS DE LA PDE10

(30) Priority: 30.06.2003 EP 03014425
(43) Date of publication of application: 05.04.2006
(73) Proprietor: Nycomed GmbH, 78467 Konstanz (DE)
(72) Inventor: BAER, Thomas, 78479 Reichenau (DE); VENNEMANN, Matthias, 78462 Konstanz (DE); MAIER, Thomas, 78333 Stockach (DE); GRAEDLER, Ulrich, 69115 Heidelberg (DE)
(74) Representative: Wild, Robert
(86) International application number: PCT/EP2004/051308
(87) International publication number: WO 2005/002579

(56) References cited:
- WO-A-02/48144
- WO-A-03/014116
- WO-A-03/051877

## Description

### Field of application of the invention

The invention relates to compounds comprising a structure-element as an integral part of the overall structure of pyrrolodihydroisoquinoline compounds, which inhibit PDE10 and can be used in the pharmaceutical industry for the production of pharmaceutical compositions.

### Known technical background

The International applications WO 02/48144, WO 03/014115, WO 03/014116, WO 03/014117 and WO 03/051877 disclose pyrrolodihydroisoquinoline derivatives with PDE10 inhibitory activity.

The European application EP 1250923 discloses the use of selective PDE10 inhibitors in general, and papaverine in particular, for the treatment of certain neurologic and psychiatric disorders.

Additionally, the US application US 2003/0008806 likewise disclose the use of selective PDE10 inhibitors in general, and papaverine in particular, for the treatment of certain neurologic and psychiatric disorders;

Yet additionally, the US application US 2003/0018047 also disclose the use of selective PDE10 inhibitors in general, and papaverine in particular, for the treatment of certain neurologic and psychiatric disorders;

The US patent US 5965575 discloses pyrrolodihydroisoquinoline derivatives as 5HT_{1B} antagonists. The International application WO 03/000269 disclose the use of PDE10A inhibitors for the treatment of neurodegenerative diseases, especially Parkinson's disease.

### Description of the invention

It has now been found that the structure-element per se, which is specified in greater details below, as well as its use as integral part of the overall structure of compounds, which inhibit PDE10, differ profoundly from prior art. Thus, said novel structure-element differs from prior art by unanticipated, sophisticated and originative structural features and its inherent, surprising and particularly advantageous function as integral part of the overall structure of compounds which inhibit PDE10.

Thus, a special aspect (special aspect A) of the present invention are compounds which comprise a structure-element of the formula X 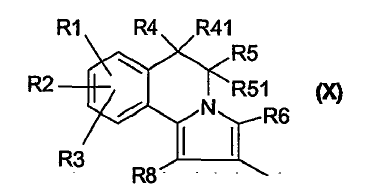 it which
R1 is halogen, nitro, 1-4C-alkyl, 1-4C-alkoxy, 1-4C-alkoxy-2-4C-alkoxy, 3-7C-cycloalkoxy or 3-7C-cycloalkylmethoxy,
R2 is 1-4C-alkoxy
R3 is hydrogen or 1-4C-alkoxy,
R4 is hydrogen,
R41 is hydrogen,
R5 is hydrogen or 1-4C-alkyl, and
R51 is hydrogen or 1-4C-alkyl,
or
R4 is hydrogen,
R41 is hydrogen,
R5 is 1-4C-alkoxycarbonyl, and
R51 is hydrogen,
or
R4 is hydrogen,
R41 is hydrogen,
R5 is cyano, and
R51 is hydrogen,
or
R4 and R5 together form a 3-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl, and
R612 is hydrogen or 1-4C-alkyl, or
R611 and R612 together and with inclusion of the nitrogen atom to which they are bound form a radical Het1, in which

Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom,
in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R8 is cyano, or -C(O)-OR9, in which
R9 is 1-4C-alkyl;
under the provisio, that,
when R8 is -C(O)-OR9, in which
R9 is 1-4C-alkyl,
then R5 is other than hydrogen;
and as integral part of the overall structure of compounds which inhibit PDE10.

An embodiment of special aspect A of the present invention are compounds which comprise a structure-element of the formula X
in which
R1 is halogen, nitro, 1-4C-alkyl, 1-4C-alkoxy, 1-4C-alkoxy-2-4C-alkoxy, 3-7C-cycloalkoxy or 3-7C-cycloalkylmethoxy,
R2 is 1-4C-alkoxy,
R3 is hydrogen or 1-4C-alkoxy,
R4 is hydrogen,
R41 is hydrogen,
R5 is hydrogen or 1-4C-alkyl, and
R51 is hydrogen or 1-4C-alkyl,
or
R4 is hydrogen,
R41 is hydrogen,
R5 is 1-4C-alkoxycarbonyl, and
R51 is hydrogen,
or
R4 and R5 together form a 3-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which R611 is hydrogen, 1-4C-alkyl, 3-7C-cycloalkyl or 3-7C-cycloalkyl-1-4C-alkyl, and
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom,
in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R8 is cyano, or -C(O)-OR9, in which
R9 is 1-4C-alkyl;
under the provisio, that,
when R8 is -C(O)-OR9, in which
R9 is 1-4C-alkyl,
then R5 is other than hydrogen;
as integral part of the overall structure of compounds which inhibit PDE10.

1-4C-Alkyl represents a straight-chain or branched alkyl radical having 1 to 4 carbon atoms. Examples which may be mentioned are the butyl, isobutyl, sec-butyl, tert-butyl, propyl, isopropyl and preferably the ethyl and methyl radicals.

2-4C-Alkyl represents a straight-chain or branched alkyl radical having 2 to 4 carbon atoms. Examples which may be mentioned are the butyl, isobutyl, sec-butyl, tert-butyl, propyl, isopropyl and preferably the ethyl radical.

1-6C-Alkyl represents a straight-chain or branched alkyl radical having 1 to 6 carbon atoms. Examples which may be mentioned are the hexyl, isohexyl (4-methylpentyl), neohexyl (3,3-dimethylbutyl), pentyl, isopentyl (3-methylbutyl), neopentyl (2,2-dimethylpropyl), butyl, isobutyl, sec-butyl, tert-butyl, propyl, isopropyl, ethyl or methyl radicals.

1-4C-Alkoxy represents radicals which, in addition to the oxygen atom, contain a straight-chain or branched alkyl radical having 1 to 4 carbon atoms. Examples which may be mentioned are the butoxy, isobutoxy, sec-butoxy, tert-butoxy, propoxy, isopropoxy and preferably the ethoxy and methoxy radicals.

2-4C-Alkoxy represents radicals which, in addition to the oxygen atom, contain a straight-chain or branched alkyl radical having 2 to 4 carbon atoms. Examples which may be mentioned are the butoxy, isobutoxy, sec-butoxy, tert-butoxy, propoxy, isopropoxy and preferably the ethoxy radical.

3-7C-Cycloalkoxy represents cyclopropyloxy, cyclobutyloxy, cyclopentyloxy, cyclohexyloxy and cycloheptyloxy, of which cyclopropyloxy, cyclobutyloxy and cyclopentyloxy are preferred.

3-7C-Cycloalkyl represents cyclopropyl, cyclobutyl, cyclopentyl, cyclohexyl and cycloheptyl, of which cyclopropyl, cyclobutyl and cyclopentyl are preferred.

3-7C-Cycloalkylmethoxy represents cyclopropylmethoxy, cyclobutylmethoxy, cyclopentylmethoxy, cyclohexylmethoxy and cycloheptylmethoxy, of which cyclopropylmethoxy, cyclobutylmethoxy and cyclopentylmethoxy are preferred.

3-7C-Cycloalkyl-1-4C-alkyl represents one of the abovementioned 1-4C-alkyl radicals, which is substituted by one of the abovementioned 3-7C-cycloalkyl radicals. Examples which may be mentioned are the cyclopropylmethyl, the cyclohexylethyl and the cyclohexylmethyl radicals.

1-4C-Alkoxy-2-4C-alkoxy represents one of the abovementioned 2-4C-alkoxy radicals, which is substituted by one of the abovementioned 1-4C-alkoxy radicals. Examples which may be mentioned are the 2-methoxyethoxy, 2-ethoxyethoxy and the 2-isopropoxyethoxy radicals.

1-4C-Alkoxy-2-4C-alkyl represents one of the abovementioned 2-4C-alkyl radicals, which is substituted by one of the abovementioned 1-4C-alkoxy radicals. Examples which may be mentioned are the 2-methoxyethyl and the 2-isopropoxyethyl radicals.

1-4C-Alkoxycarbonyl represents a radical which, in addition to the carbonyl group, contains one of the abovementioned 1-4C-alkoxy radicals. Examples which may be mentioned are the methoxycarbonyl and ethoxycarbonyl radicals.

3-4C-Alkylene is a straight-chain alkylene radical such as, for example, the trimethylene (-CH₂-CH₂-CH₂-) or the tetramethylene (-CH₂-CH₂-CH₂-CH₂-) radical.

Halogen within the meaning of the invention is bromine and, preferably, chlorine and fluorine.

Hydroxy-2-4C-alkyl stands for one of the abovementioned 2-4C-alkyl radicals which is substituted by a hydroxyl group. Examples which may be mentioned are the 2-hydroxyethyl and 3-hydroxypropyl radicals.

Amino-2-4C-alkyl stands for one of the abovementioned 2-4C-alkyl radicals which is substituted by an amino group. Examples which may be mentioned are the 2-aminoethyl and 3-aminopropyl radicals.

In addition to the nitrogen atom, mono- or di-1-4C-alkylamino radicals contain one or two of the abovementioned 1-4C-alkyl radicals. Di-1-4C-alkylamino is to be emphasized and here, in particular, dimethyl-, diethyl- and diisopropylamino.

Mono- or Di-1-4C-alkylamino-2-4C-alkyl stands for one of the abovementioned 2-4C-alkyl radicals which is substituted by one of the abovementioned mono- or di-1-4C-alkylamino groups. Examples which may be mentioned are the 2-dimethylaminoethyl and 3-dimethylaminopropyl radicals.

Het1 refers to a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom. Examples for Het2 include e.g. piperidin-1-yl, 4-methyl-piperidin-1-yl, 4-hydroxypiperidin-1-yl, morpholin-4-yl, pyrrolidin-1-yl, piperazin-1-yl, imidazolidin-1-yl, thiomorpholin-4-yl, homopiperidin-1-yl, homopiperazin-1-yl, 4-N-(1-4C-alkyl)-homopiperazin-1-yl or piperazinyl substituted on a ring nitrogen atom by R613 [4-N-(R613)-piperazin-1-yl] such as, for example, 4-N-(1-4C-alkyl)-piperazin-1-yl, 4-N-(hydroxy-2-4C-alkyl)-piperazin-1-yl, 4-N-(dimethylamino-2-4C-alkyl)-piperazin-1-yl, 4-N-(3-6C-cycloalkyl)-piperazin-1-yl, 4-N-formyl-piperazin-1-yl, 4-N-(pyridin-4-yl)-piperazin-1-yl, 4-N-(pyrimidin-2-yl)-piperazin-1-yl or 4-N-(3-6C-cycloalkylmethyl)-piperazin-1-yl.

N-(1-4C-alkyl)-piperazinyl stands for the piperaan-1-yl radical substituted by one of the abovementioned 1-4C-alkyl radicals on the 4-N ring nitrogen atom.

Constituents which are substituted as described herein may be substituted, unless otherwise noted, at any possible position.

Thus, the substituents R1, R2 and/or R3 may be attached, unless otherwise noted, at any position of the benzo moiety of the pyrrolodihydroisoquinoline ring.

A subaspect (subaspect a1) of the special aspect a according to this invention worthy to be mentioned are compounds which comprise a structure-element of the formula X,
in which
R1 is halogen, nitro, 1-4C-alkyl, 1-4C-alkoxy, 1-4C-alkoxy-2-4C-alkoxy, 3-7C-cycloalkoxy or 3-7C-cycloalkylmethoxy,
R2 is 1-4C-alkoxy
R3 is hydrogen or 1-4C-alkoxy,
R4 is hydrogen,
R41 is hydrogen,
R5 is ethyl or, in particular, methyl,
R51 is hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl,
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom,
in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R8 is cyano,
and which inhibit PDE10.

A subaspect (subaspect a2) of the special aspect A according to this invention more worthy to be mentioned are compounds which comprise a structure-element of the formula X,
in which
- R1: is halogen or 1-4C-alkoxy,
- R2: is hydrogen, or 1-4C-alkoxy,
- R3: is 1-4C-alkoxy,
- R4: is hydrogen,
- R41: is hydrogen,
- R5: is hydrogen, methyl or ethyl,
- R51: is hydrogen,
- R6: is methyl, ethyl or methoxycarbonylethyl,
- R8: is cyano,
and which inhibit PDE10.

A subaspect (subaspect a3) of the special aspect A according to this invention in particular worthy to be mentioned are compounds which comprise a structure-element of the formula Xa or Xb 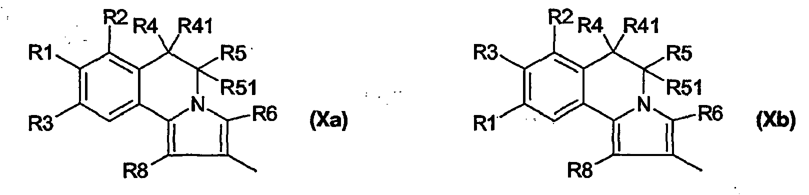 in which
as a first alternative,
- R1: is chlorine or fluorine,
- R2: is hydrogen,
- R3: is methoxy or ethoxy,
or, as a second alternative,
- R1: is methoxy or ethoxy,
- R2: is hydrogen,
- R3: is methoxy or ethoxy,
or, as a third alternative,
- R1: is chlorine or fluorine,
- R2: is methoxy or ethoxy,
- R3: is methoxy or ethoxy,
or, as a fourth alternative,
- R1: is methoxy or ethoxy,
- R2: is chlorine or fluorine,
- R3: is methoxy or ethoxy,
or, as a fifth alternative,
- R1: is methoxy or ethoxy,
- R2: is methoxy or ethoxy,
- R3: is methoxy or ethoxy,
- R4: is hydrogen,
- R41: is hydrogen,
- R5: is ethyl or, in particular, methyl,
- R51: is hydrogen,
- R6: is methyl, ethyl or methoxycarbonylethyl,
- R8: is cyano,
and which inhibit PDE10.

A subaspect (subaspect a4) of the special aspect A according to this invention in more particular worthy to be mentioned are compounds which comprise a structure-element of the formula Xa,
in which
- R1: is methoxy,
- R2: is hydrogen,
- R3: is methoxy,
- R4: is hydrogen,
- R41: is hydrogen,
- R5: is methyl,
- R51: is hydrogen,
- R6: is methyl or methoxycarbonylethyl,
- R8: is cyano,
as integral part of the overall structure of compounds which inhibit PDE10.

Another subaspect (subaspect b1) of the special aspect A according to this invention worthy to be mentioned is the use of a structure-element of the formula X,
in which
- R1: is halogen or 1-4C-alkoxy,
- R2: is hydrogen, or 1-4C-alkoxy,
- R3: is 1-4C-alkoxy,
- R4: is hydrogen,
- R41: is hydrogen,
- R5: is methyl or ethyl,
- R51: is hydrogen,
- R6: is methyl, ethyl or methoxycarbonylethyl,
- R8: is -C(O)-OR9, in which
- R9: is 1-4C-alkyl,
and which inhibit PDE10.

A further subaspect (subaspect b2) of the special aspect A according to this invention more worthy to be mentioned are the compounds which comprise a structure-element of the formulae Xa or Xb,
in which
as a first alternative,
- R1: is chlorine or fluorine,
- R2: is hydrogen,
- R3: is methoxy or ethoxy,
or, as a second alternative,
- R1: is methoxy or ethoxy,
- R2: is hydrogen,
- R3: is methoxy or ethoxy,
or, as a third alternative,
- R1: is chlorine or fluorine,
- R2: is methoxy or ethoxy,
- R3: is methoxy or ethoxy,
or, as a fourth alternative,
- R1: is methoxy or ethoxy,
- R2: is chlorine or fluorine,
- R3: is methoxy or ethoxy,
or, as a fifth alternative,
- R1: is methoxy or ethoxy,
- R2: is methoxy or ethoxy,
- R3: is methoxy or ethoxy,
- R4: is hydrogen,
- R41: is hydrogen,
- R5: is ethyl or, in particular, methyl,
- R51: is hydrogen,
- R6: is methyl, ethyl or methoxycarbonylethyl,
- R8: is -C(O)-OR9, in which
- R9: is 1-4C-alkyl,
and which inhibit PDE10.

A further subaspect (subaspect b3) of the special aspect A according to this invention in particular worthy to be mentioned are the compounds which comprise a structure-element of the formula Xa,
in which
- R1: is methoxy,
- R2: is hydrogen,
- R3: is methoxy,
- R4: is hydrogen,
- R41: is hydrogen,
- R5: is methyl,
- R51: is hydrogen,
- R6: is methyl or methoxycarbonylethyl,
- R8: is -C(O)-OR9, in which
- R9: is 1-4C-alkyl,
and which inhibit PDE10.

Regarding the aforementioned subaspects according to the present invention, the subaspects a1, a2, a3 and a4 are to be emphasized.
The subaspects a2, a3 and a4 are in particular to be emphasized, and the subaspects a3 and, especially, a4 are in more particular to be emphasized.

Another subaspect (subaspect c1) of the special aspect A according to this invention worthy to be mentioned are the compounds which comprise a structure-element of the formula Xa or Xb,
in which
- R1: is 1-4C-alkoxy, 1-4C-alkoxy-2-4C-alkoxy, 3-7C-cycloalkoxy or 3-7C-cycloalkylmethoxy,
- R2: is hydrogen
- R3: is 1-4C-alkoxy,
- R4: is hydrogen,
- R41: is hydrogen,
- R5: is 1-4C-alkyl or cyano,
- R51: is hydrogen,
- R6: is 1-4C-alkyl, or 1-4C-alkyl substituted by 1-4C-alkoxycarbonyl,
- R8: is -C(O)-OR9, in which
- R9: is 1-4C-alkyl,
and which inhibit PDE10.

A further subaspect (subaspect c2) of the special aspect A according to this invention more worthy to be mentioned are the compounds which comprise a structure-element of the formula Xa or Xb,
in which
- R1: is 1-4C-alkoxy,
- R2: is hydrogen
- R3: is 1-4C-alkoxy,
- R4: is hydrogen,
- R41: is hydrogen,
- R5: is 1-4C-alkyl or cyano,
- R51: is hydrogen,
- R6: is 1-4C-alkyl, or 1-4C-alkyl substituted by 1-4C-alkoxycarbonyl,
- R8: is -C(O)-OR9, in which
- R9: is 1-4C-alkyl,
and which inhibit PDE10.

A further subaspect (subaspect c3) of the special aspect A according to this invention more worthy to be mentioned are the compounds which comprise a structure-element of the formula Xa or Xb,
in which
- R1: is 1-2C-alkoxy,
- R2: is hydrogen,
- R3: is 1-2C-alkoxy,
- R4: is hydrogen,
- R41: is hydrogen,
- R5: is 1-2C-alkyl or cyano,
- R51: is hydrogen,
- R6: is 1-2C-alkyl, or 1-2C-alkyl substituted by 1-2C-alkoxycarbonyl,
- R8: is -C(O)-OR9, in which
- R9: is 1-2C-alkyl,
and which inhibit PDE10.

Another subaspect (subaspect d1) of the special aspect A according to this invention worthy to be mentioned are the compounds which comprise a structure-element of the formula Xa or Xb,
in which
- R1: is 1-4C-alkoxy, 1-4C-alkoxy-2-4C-alkoxy, 3-7C-cycloalkoxy or 3-7C-cycloalkylmethoxy,
- R2: is hydrogen
- R3: is 1-4C-alkoxy,
- R4: is hydrogen,
- R41: is hydrogen,
- R5: is hydrogen, 1-4C-alkyl or cyano,
- R51: is hydrogen,
- R6: is 1-4C-alkyl, or 1-4C-alkyl substituted by 1-4C-alkoxycarbonyl,
- R8: is cyano,
and which inhibit PDE10.

A further subaspect (subaspect d2) of the special aspect A according to this invention more worthy to be mentioned are the compounds which comprise a structure-element of the formula Xa or Xb,
in which
- R1: is 1-4C-alkoxy,
- R2: is hydrogen,
- R3: is 1-4C-alkoxy,
- R4: is hydrogen,
- R41: is hydrogen,
- R5: is hydrogen, 1-4C-alkyl or cyano,
- R51: is hydrogen,
- R6: is 1-4C-alkyl, or 1-4C-alkyl substituted by 1-4C-alkoxycarbonyl,
- R8: is cyano,
and which inhibit PDE10.

A further subaspect (subaspect d3) of the special aspect A according to this invention further more worthy to be mentioned are the compounds which comprise a structure-element of the formula Xa or Xb,
in which
- R1: is 1-2C-alkoxy,
- R2: is hydrogen,
- R3: is 1-2C-alkoxy,
- R4: is hydrogen,
- R41: is hydrogen,
- R5: is hydrogen, 1-2C-alkyl or cyano,
- R51: is hydrogen,
- R6: is 1-2C-alkyl, or 1-2C-alkyl substituted by 1-2C-alkoxycarbonyl,
- R8: is cyano,
and which inhibit PDE10.

A further subaspect (subaspect d4) of the special aspect A according to this invention still further more worthy to be mentioned are the compounds which comprise a structure-element of the formula Xa or Xb,
in which
- R1: is 1-2C-alkoxy,
- R2: is hydrogen,
- R3: is 1-2C-alkoxy,
- R4: is hydrogen,
- R41: is hydrogen,
- R5: is 1-2C-alkyl or cyano,
- R51: is hydrogen,
- R6: is 1-2C-alkyl, or 2-methoxycarbonylethyl,
- R8: is cyano,
and which inhibit PDE10.

A further subaspect (subaspect d6) of the special aspect A according to this invention still yet further more worthy to be mentioned are the compounds which comprise a structure-element of the formula Xa or Xb,
in which
- R1: is methoxy,
- R2: is hydrogen,
- R3: is methoxy,
- R4: is hydrogen,
- R41: is hydrogen,
- R5: is methyl or cyano,
- R51: is hydrogen,
- R6: is methyl, or 2-methoxycarbonylethyl,
- R8: is cyano,
and which inhibit PDE10.

Within the meaning of this invention, a particular subaspect (subaspect e1) of the special aspect A of this invention are the compounds which comprise a structure-element selected from the group consisting of those structure-elements of the formula Xa,
in which
- R1: is methoxy,
- R3: is methoxy,
- R4: is hydrogen,
- R41: is hydrogen,
- R51: is hydrogen,
and in which the following combinations of the substituent meanings for R2, R5, R6 and R8 of Table 1 apply:

**Table 1:**

| | R2 | R5 | R6 | R8 |
|---|---|---|---|---|
| 1.) | hydrogen | methyl | methyl | cyano |
| 2.) | hydrogen | methyl | methyl | ethoxycarbonyl |
| 3.) | hydrogen | methyl | 2-methoxycarbonylethyl | cyano |
| 4.) | hydrogen | methyl | 2-methoxycarbonylethyl | ethoxycarbonyl |
| 5.) | hydrogen | hydrogen | methyl | cyano |
| 6.) | hydrogen | hydrogen | 2-methoxycarbonylethyl | cyano |
| 15.) | hydrogen | cyano | methyl | cyano |
| 16.) | hydrogen | cyano | methyl | ethoxycarbonyl |
| 17.) | hydrogen | cyano | 2-methoxycarbonylethyl | cyano |
| 18.) | hydrogen | cyano | 2-methoxycarbonylethyl | ethoxycarbonyl |
| 35.) | hydrogen | methyl | methyl | methoxycarbonyl |
| 36.) | hydrogen | methyl | 2-methoxycarbonylethyl | methoxycarbonyl |
| 41.) | hydrogen | cyano | methyl | methoxycarbonyl |
| 42.) | hydrogen | cyano | 2-methoxycarbonylethyl | methoxycarbonyl |

whereby the combinations 1 to 6, 16, and 35 are to be emphasized, and
whereby the combinations 1, 3, 5, 6 are to be more emphasized and which inhibit PDE10.

Originally filed combinations 7-14, 19-34, 37-40, 43-50 of Table 1 have been abandoned.

A further aspect of the present invention are the compounds which comprise a structure-element of the formula X, Xa or Xb, in which R1, R2, R3, R4, R41, R5, R51, R6 and R8 have the meanings given above in the special aspect A, in particular in the subaspects thereto, in more particular in the subaspects emphasized above, which inhibit effectively PDE10, for use in treating disorders of the central nervous system.

A further aspect of the present invention are the compounds which comprise a structure-element of the formula X, Xa or Xb, in which R1, R2, R3, R4, R41, R5, R51, R6 and R8 have the meanings given above in the special aspect A, particularly, in the subaspects thereto, as of compounds for use in therapy.

A further aspect of the present invention are the compounds which comprise a structure-element of the formula X, Xa or Xb, in which R1, R2, R3, R4, R41, R5, R51, R6 and R8 have the meanings given above in the special aspect A, particularly, in the subaspects thereto, which inhibit PDE10, for use in the manufacture of pharmaceutical compositions.

A further aspect of the present invention the provision of compounds which comprise a structure-element of the formula X, Xa or Xb, in which R1, R2, R3, R4, R41, R5, R51, R6 and R8 have the meanings given above in the special aspect A, particularly, in the subaspects thereto, to provide compounds, which inhibit PDE10.

Also disclosed is a process to provide compounds, which inhibit PDE10, comprising the following steps:
a.) designing intellectually the structure of a compound comprising - as part of its overall structure - a structure-element of the formula X, Xa or Xb, in which R1, R2, R3, R4, R41, R5, R51, R6 and R8 have the meanings given above in the special aspect A, in particular, in the subaspects thereto, in more particular in the subaspects emphasized above;
b.) synthesizing materially a compound having said designed structure in a manner known to the person skilled in the art, or as disclosed in the specification of the present invention, or as disclosed in WO 02/48144, WO 03/014115, WO 03/014116 or WO 03/014117, or analogously or similarly thereto.

A further aspect of the present invention is a compound obtainable by the abovementioned process to provide compounds, which inhibit PDE10.

A further aspect of the present invention are compounds which inhibit effectively PDE10 and which comprise a structure-element of the formula X, Xa or Xb, in which R1, R2, R3, R4, R41, R5, R51, R6 and R8 have the meanings given above in the special aspect A, particularly, in the subaspects thereto, as an integral part of their overall structures.

Consequentially, compounds according to the present invention include those compounds, which comprise a structure-element of the formula X, Xa or Xb, in which R1, R2, R3, R4, R41, R5, R51, R6 and R8 have the meanings given above in the special aspect A.

A further aspect of the present invention is a process or method for providing PDE10 inhibitors of the pyrrolodihydroisoquinoline class with advantageous pharmacological properties (e.g. improved effectiveness) comprising the following steps:
a.) selecting intellectually a structure of a compound of the pyrrolodihydroisoquinoline class;
b.) modifying intellectually said selected structure in such a way that the modified structure comprises - as part of its overall structure - a structure element of the formula X, Xa or Xb, in which R1, R2, R3, R4, R41, R5, R51, R6 and R8 have the meanings given above in the special aspect A, in particular, in the subaspects thereto, in more particular in the subaspects emphasized above;
c.) synthesizing materially a compound having said modified structure in a manner known to the person skilled in the art, or as disclosed in the specification of the present invention, or as disclosed in WO 02/48144, WO 03/014115, WO 03/014116 or WO 03/014117, or analogously or similarly thereto.

Also disclosed is a method for providing PDE10 inhibitors of the pyrrolodihydroisoquinoline class with advantageous properties comprising the following steps:
a.) selecting intellectually a structure of a compound of the pyrrolodihydroisoquinoline class, or, advantageously,
   selecting intellectually a structure of a compound disclosed, in a first embodiment, in WO 02/48144, WO 03/014115, WO 03/014116 or WO 03/014117, or, in a second embodiment, in WO 02/48144, WO 03/014115, WO 03/014116, WO 03/014117 or WO 03/051877,
   preferably a structure of a compound mentioned expressis verbis or individualized by way of example therein,
   more preferably a structure of a compound emphasized therein and/or a structure of a compound disclosed therein with advantageous effects (e.g. advantageous PDE10 inhibiting values);
b.) modifying intellectually said selected structure in such a way that the modified structure comprises - as part of its overall structure - a structure element of the formula X, Xa or Xb, in which R1, R2, R3, R4, R41, R5, R51, R6 and R8 have the meanings given above in the special aspect A, in particular, in the subaspects thereto, in more particular in the subaspects emphasized above;
c.) synthesizing materially a compound having said modified structure in a manner known to the person skilled in the art, or as disclosed in the specification of the present invention, or as disclosed, in a first embodiment, in WO 02/48144, WO 03/014115, WO 03/014116 or WO 03/014117, or, in a second embodiment, in WO 02/48144, WO 03/014115, WO 03/014116, WO 03/014117 or WO 03/051877,
or analogously or similarly thereto.

A further aspect of the present invention is a compound obtainable by one of the abovementioned processes or methods for providing PDE10 inhibitors of the pyrrolodihydroisoquinoline class with advantageous properties.

A further aspect of the present invention are the compounds which comprise a structure-element of the formula X, Xa or Xb, in which R1, R2, R3, R4, R41, R5, R51, R6 and R8 have the meanings given above in the special aspect A, in particular in the subaspects thereto, in more particular in the subaspects emphasized above, as an integral part of the overall structure of compounds, which are suitable to inhibit PDE10, and which have a molecular weight between 200 and 600 daltons.

As exemplary compounds according to this invention may be mentioned any of those compounds individualized and listed as Examples 1 to 28 in the following examples, or a salt, stereoisomer, hydrate or hydrate of a salt thereof.

As further exemplary compounds according to this invention may be mentioned any compound selected from the group consisting of those compounds individualized and listed as Examples 29 to 69 in the following examples, or a salt, stereoisomer, hydrate or hydrate of a salt thereof.

As exemplary compounds according to this invention the following compounds of formula Ic, and the salts, stereoisomers, hydrates or hydrates of the salts thereof, 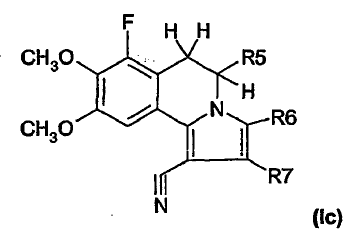 may be notably mentioned by means of the substituent meanings for R5, R6 and R7 in the following Tables A1, A2, A3 and A4:

**Table A1:**

| Example No. | R5 | R6 | R7 |
|---|---|---|---|
| 70. | -CH₃ | -CH₃ | 4-methoxy-3,5-dimethylphenyl |
| 71. | -CH₃ | -CH₃ | 4-carboxy-phenyl |
| 72. | -CH₃ | -CH₃ | 2-methyl-4-hydroxy-phenyl |
| 73. | -CH₃ | -CH₃ | 4-amino-phenyl |
| 74. | -CH₃ | -CH₃ | 4-(2H-tetrazol-5-yl)-phenyl |
| 75. | -CH₃ | -CH₃ | 4-morpholino-sulphonylamino-phenyl |
| 76. | -CH₃ | -CH₃ | 4-methylsulphonylamino-phenyl |
| 77. | -CH₃ | -CH₃ | pyridin-4-yl |
| 78. | -CH₃ | -CH₃ | quinolin-4-yl |
| 79. | -CH₃ | -CH₃ | 2-methyl-pyridin-4-yl |
| 80. | -CH₃ | -CH₃ | 3-methyl-pyridin-4-yl |
| 81. | -CH₃ | -CH₃ | 1-tolylsulphonyl-pyrrol-3-yl |
| 82. | -CH₃ | -CH₃ | 1-phenylsulphonyl-indol-3-yl |
| 83. | -CH₃ | -CH₃ | 1-methylsulphonyl-indol-3-yl |
| 84. | -CH₃ | -CH₃ | 1-dimethylaminosulphonyl-indol-3-yl |
| 85. | -CH₃ | -CH₃ | 1-morpholinosulphonyl-indol-3-yl |

**Table A2:**

| Example No. | R5 | R6 | R7 |
|---|---|---|---|
| 86. | -CN | -CH₃ | 4-hydroxy-3,5-dimethylphenyl |
| 87. | -CN | -CH₃ | 4-methoxy-3,5-dimethylphenyl |
| 88. | -CN | -CH₃ | 4-carboxy-phenyl |
| 89. | -CN | -CH₃ | 2-methyl-4-hydroxy-phenyl |
| 90. | -CN | -CH₃ | 4-amino-phenyl |
| 91 | -CN | -CH₃ | 4-(2H-tetrazol-5-yl)-phenyl |
| 92. | -CN | -CH3 | 4-morpholino-sulphonylamino-phenyl |
| 93. | -CN | -CH₃ | 4-methylsulphonylamino-phenyl |
| 94. | -CN | -CH₃ | pyridin-4-yl |
| 95. | -CN | -CH₃ | quinolin-4-yl |
| 96. | -CN | -CH₃ | 2-methyl-pyridin-4-yl |
| 97. | -CN | -CH₃ | 3-methyl-pyridin-4-yl |
| 98. | -CN | -CH₃ | 1-tolylsulphonyl-pyrrol-3-yl |
| 99. | -CN | -CH₃ | 1-tolylsulphonyl-indol-3-yl |
| 100. | -CN | -CH₃ | 1-phenylsulphonyl-indol-3-yl |
| 101. | -CN | -CH₃ | 1-methylsulphonyl-indol-3-yl |
| 102. | -CN | -CH₃ | 1-dimethylaminosulphonyl-indol-3-yl |
| 103. | -CN | -CH₃ | 1-morpholinosulphonyl-indol-3-yl |

**Table A3:**

| Example No. | R5 | R6 | R7 |
|---|---|---|---|
| 104. | -CH₃ | -(CH₂CH₂)C(O)OCH₃ | 4-hydroxy-3,5-dimethylphenyl |
| 105. | -CH₃ | -(CH₂CH₂)C(O)OCH₃ | 4-methoxy-3,5-dimethylphonyl |
| 106. | -CH₃ | -(CH₂CH₂)C(O)OCH₃ | 4-carboxy-phenyl |
| 107. | -CH₃ | -(CH₂CH₂)C(O)OCH₃ | 2-methyl-4-hydroxy-phenyl |
| 108. | -CH₃ | -(CH₂CH₂)C(O)OCH₃ | 4-amino-phenyl |
| 109. | -CH₃ | -(CH₂CH₂)C(O)OCH₃ | 4-(2H-tetrazol-5-yl)-phenyl |
| 110. | -CH₃ | -(CH₂CH₂)C(O)OCH₃ | 4-morpholino-sulphonylamino-phenyl |
| 111. | -CH₃ | -(CH₂CH₂)C(O)OCH₃ | 4-methylsulphonylamino-phenyl |
| 112. | -CH₃ | -(CH₂CH₂)C(O)OCH₃ | pyridin-4-yl |
| 113. | -CH₃ | -(CH₂CH₂)C(O)OCH₃ | quinolin-4-yl |
| 114. | -CH₃ | -(CH₂CH₂)C(O)OCH₃ | 2-methyl-pyridin-4-yl |
| 115. | -CH₃ | -(CH₂CH₂)C(O)OCH₃ | 3-mdthyl-pyridin-4-yl |
| 116. | -CH₃ | -(CH₂CH₂)C(O)OCH₃ | 1-tolylsulphonyl-pyrrol-3-yl |
| 117. | -CH₃ | -(CH₂CH₂)C(O)OCH₃ | 1-tolylsulphonyl-indol-3-yl |
| 118. | -CH₃ | -(CH₂CH₂)C(O)OCH₃ | 1-phenylsulphonyl-indol-3-yl |
| 119. | -CH₃ | -(CH₂CH₂)C(O)OCH₃ | 1-methylsulphonyl-indol-3-yl |
| 120. | -CH₃ | -(CH₂CH₂)C(O)OCH₃ | 1-dimethylaminosulphonyl-indol-3-yl |
| 121. | -CH₃ | -(CH₂CH₂)C(O)OCH₃ | 1-morpholinosulphonyl-indol-3-yl |

**Table A4:**

| Example No. | R5 | R6 | R7 |
|---|---|---|---|
| 122. | -CN | -CH₃ | 4-hydroxy-3,5-dimethylphenyl |
| 123. | -CN | -CH₃ | 4-methoxy-3,5-dimethylphenyl |
| 124. | -CN | -CH₃ | 4-carboxy-phenyl |
| 125. | -CN | -CH₃ | 2-methyl-4-hydroxy-phenyl |
| 126. | -CN | -CH₃ | 4-amino-phenyl |
| 127. | -CN | -CH₃ | 4-(2H-tetrazol-5-yl)-phenyl |
| 128. | -CN | -CH₃ | 4-morpholino-sulphonylamino-phenyl |
| 129. | -CN | -CH₃ | 4-methylsulphonylamino-phenyl |
| 130. | -CN | -CH₃ | pyridin-4-yl |
| 131. | -CN | -CH₃ | quinolin-4-yl |
| 132. | -CN | -CH₃ | 2-methyl-pyridin-4-yl |
| 133. | -CN | -CH₃ | 3-methyl-pyridin-4-yl |
| 134. | -CN | -CH₃ | 1-tolylsulphonyl-pyrrol-3-yl |
| 135. | -CN | -CH₃ | 1-tolylsulphonyl-indol-3-yl |
| 136. | -CN | -CH₃ | 1-phenylsulphonyl-indol-3-yl |
| 137. | -CN | -CH₃ | 1-methylsulphonyl-indol-3-yl |
| 138. | -CN | -CH₃ | 1-dimethylaminosulphonyl-indol-3-yl |
| 139. | -CN | -CH₃ | 1-morpholinosulphonyl-indol-3-yl |

as well as compound 30 and compound 42.

The following compounds of formula Id or Ie, and the salts, stereoisomers, hydrates or hydrates of the salts thereof, 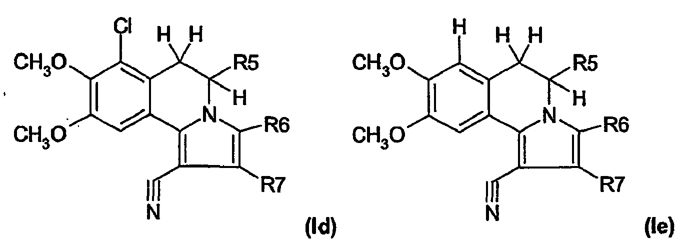 may be also mentioned as exemplary compounds according to this invention by means of the substituent meanings for R5, R6 and R7 in the following Tables B1, B2, B3 and B4:

**Table B1:**

| R5 | R6 | R7 |
|---|---|---|
| -CH₃ | -CH₃ | 4-hydroxy-3,5-dimethylphenyl |
| -CH₃ | -CH₃ | 4-methoxy-3,5-dimethylphenyl |
| -CH₃ | -CH₃ | 4-carboxy-phenyl |
| -CH₃ | -CH₃ | 2-methyl-4-hydroxy-phenyl |
| -CH₃ | -CH₃ | 4-amino-phenyl |
| -CH₃ | -CH₃ | 4-(2H-tetrazol-5-yl)-phenyl |
| -CH₃ | -CH₃ | 4-morpholino-sulphonylamino-phenyl |
| -CH₃ | -CH₃ | 4-methylsulphonylamino-phenyl |
| -CH₃ | -CH₃ | pyridin-4-yl |
| -CH₃ | -CH₃ | quinolin-4-yl |
| -CH₃ | -CH₃ | 2-methyl-pyridin-4-yl |
| -CH₃ | -CH₃ | 3-methyl-pyridin-4-yl |
| -CH₃ | -CH₃ | 1-tolylsulphonyl-pyrrol-3-yl |
| -CH₃ | -CH₃ | 1-phenylsulphonyl-indol-3-yl |
| -CH₃ | -CH₃ | 1-methylsulphonyl-indol-3-yl |
| -CH₃ | -CH₃ | 1-dimethylaminosulphonyl-indol-3-yl |
| -CH₃ | -CH₃ | 1-morpholinosulphonyl-indol-3-yl |

**Table B2:**

| R5 | R6 | R7 |
|---|---|---|
| -CN | -CH₃ | 4-hydroxy-3,5-dimethylphenyl |
| -CN | -CH₃ | 4-methoxy-3,5-dimethylphenyl |
| -CN | -CH₃ | 4-carboxy-phenyl |
| -CN | -CH₃ | 2-methyl-4-hydroxy-phenyl |
| -CN | -CH₃ | 4-amino-phenyl |
| -CN | -CH₃ | 4-(2H-tetrazol-5-yl)-phenyl |
| -CN | -CH₃ | 4-morpholino-sulphonylamino-phenyl |
| -CN | -CH₃ | 4-methylsulphonylamino-phenyl |
| -CN | -CH₃ | pyridin-4-yl |
| -CN | -CH₃ | quinolin-4-yl |
| -CN | -CH₃ | 2-methyl-pyridin-4-yl |
| -CN | -CH₃ | 3-methyl-pyridin-4-yl |
| -CN | -CH₃ | 1-tolylsulphonyl-pyrrol-3-yl |
| -CN | -CH₃ | 1-tolylsulphonyl-indol-3-yl |
| -CN | -CH₃ | 1-phenylsulphonyl-indol-3-yl |
| -CN | -CH₃ | 1-methylsulphonyl-indol-3-yl |
| -CN | -CH₃ | 1-dimethylaminosulphonyl-indol-3-yl |
| -CN | -CH₃ | 1-morpholinosulphonyl-indol-3-yl |

**Table B3:**

| R5 | R6 | R7 |
|---|---|---|
| -CH₃ | -(CH₂CH₂)C(O)OCH₃ | 4-hydroxy-3,5-dimethylphenyl |
| -CH₃ | -(CH₂CH₂)C(O)OCH₃ | 4-methoxy-3,5-dimethylphenyl |
| -CH₃ | -(CH₂CH₂)C(O)OCH₃ | 4-carboxy-phenyl |
| -CH₃ | -(CH₂CH₂)C(O)OCH₃ | 2-methyl-4-hydroxy-phenyl |
| -CH₃ | -(CH₂CH₂)C(O)OCH₃ | 4-amino-phenyl |
| -CH₃ | -(CH₂CH₂)C(O)OCH₃ | 4-(2H-tetrazol-5-yl)-phenyl |
| -CH₃ | -(CH₂CH₂)C(O)OCH₃ | 4-morpholino-sulphonylamino-phenyl |
| -CH₃ | -(CH₂CH₂)C(O)OCH₃ | 4-methylsulphonylamino-phenyl |
| -CH₃ | -(CH₂CH₂)C(O)OCH₃ | pyridin-4-yl |
| -CH₃ | -(CH₂CH₂)C(O)OCH₃ | quinolin-4-yl |
| -CH₃ | -(CH₂CH₂)C(O)OCH₃ | 2-methyl-pyridin-4-yl |
| -CH₃ | -(CH₂CH₂)C(O)OCH₃ | 3-methyl-pyridin-4-yl |
| -CH₃ | -(CH₂CH₂)C(O)OCH₃ | 1-tolylsulphonyl-pyrrol-3-yl |
| -CH₃ | -(CH₂CH₂)C(O)OCH₃ | 1-tolylsulphonyl-indol-3-yl |
| -CH₃ | -(CH₂CH₂)C(O)OCH₃ | 1-phenylsulphonyl-indol-3-yl |
| -CH₃ | -(CH₂CH₂)C(O)OCH₃ | 1-methylsulphonyl-indol-3-yl |
| -CH₃ | -(CH₂CH₂)C(O)OCH₃ | 1-dimethylaminosulphonyl-indol-3-yl |
| -CH₃ | -(CH₂CH₂)C(O)OCH₃ | 1-morpholinosulphonyl-indol-3-yl |

**Table B4:**

| R5 | R6 | R7 |
|---|---|---|
| -CN | -CH₃ | 4-hydroxy-3,5-dimethylphenyl |
| -CN | -CH₃ | 4-methoxy-3,5-dimethylphenyl |
| -CN | -CH₃ | 4-carboxy-phenyl |
| -CN | -CH₃ | 2-methyl-4-hydroxy-phenyl |
| -CN | -CH₃ | 4-amino-phenyl |
| -CN | -CH₃ | 4-(2H-tetrazol-5-yl)-phenyl |
| -CN | -CH₃ | 4-morpholino-sulphonylamino-phenyl |
| -CN | -CH₃ | 4-methylsulphonylamino-phenyl |
| -CN | -CH₃ | pyridin-4-yl |
| -CN | -CH₃ | quinolin-4-yl |
| -CN | -CH₃ | 2-methyl-pyridin-4-yl |
| -CN | -CH₃ | 3-methyl-pyridin-4-yl |
| -CN | -CH₃ | 1-tolylsulphonyl-pyrrol-3-yl |
| -CN | -CH₃ | 1-tolylsulphonyl-indol-3-yl |
| -CN | -CH₃ | 1-phenylsulphonyl-indol-3-yl |
| -CN | -CH₃ | 1-methylsulphonyl-indol-3-yl |
| -CN | -CH₃ | 1-dimethylaminosulphonyl-indol-3-yl |
| -CN | -CH₃ | 1-morpholinosulphonyl-indol-3-yl |

The compounds which comprise a structure-element of the formula X, Xa or Xb, in which R1, R2, R3, R4, R41, R5, R51, R6 and R8 have the meanings given above in the special aspect A, can be prepared, for example, in an art-known manner, or in a manner described and shown as follows, or as disclosed in WO 02/48144, WO 03/014115, WO 03/014116, WO 03/014117 or WO 03/051877, or as described by way of example in the following examples, or analogously or similarly thereto.

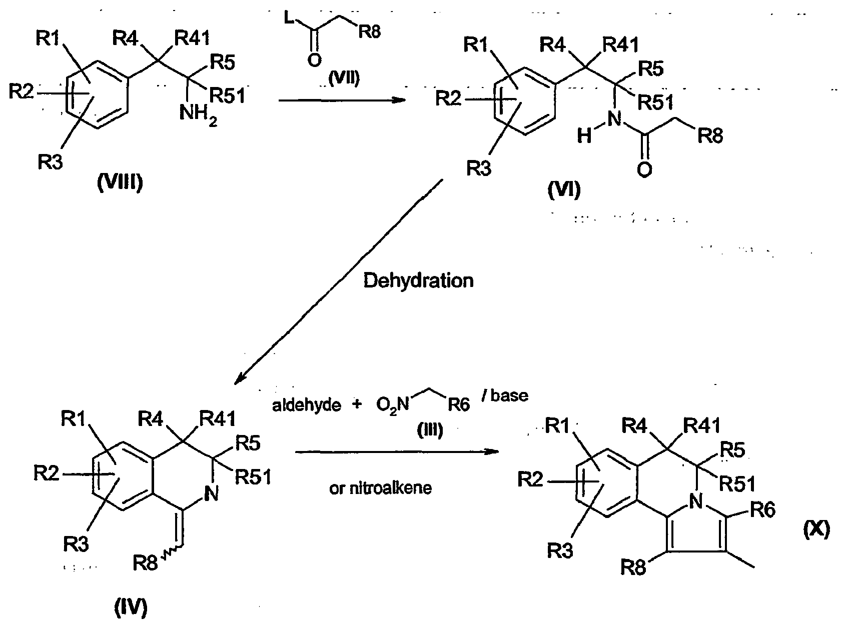

As shown in the scheme above, in a first reaction step compounds of formula VIII, in which R1, R2, R3, R4, R41, R5 and R51 have the meanings indicated above, are reacted with compounds of formula VII, in which R8 has the meanings indicated above and L is a suitable leaving group, for example chlorine or a acyloxy radical (e.g. the R8-CH₂-C(O)-O- radical), to give in the presence of a suitable organic or inorganic base corresponding compounds of formula VI.

Alternatively, compounds of formula VI are also accessible from compounds of formula VIII, in which R1, R2, R3, R4, R41, R5 and R51 have the meanings indicated above, and compounds of formula VII, in which R8 has the meanings indicated above and L is hydroxyl, by reaction with amide bond linking reagents known to the person skilled in the art. Exemplary amide bond linking reagents known to the person skilled in the art which may be mentioned are, for example, the carbodiimides (e.g. dicyclohexylcarbodiimide or, preferably, 1-ethyl-3-(3-dimethylaminopropyl)carbodiimide hydrochloride), azodicarboxylic acid derivatives (e.g. diethyl azodicarboxylate), uronium salts [e.g. O-(benzotriazol-1-yl)-N,N,N',N'-tetramethyluronium tetrafluoroborate or O-(benzotriazol-1yl)-N,N,N',N'-tetramthyl-uronium-hexafluorophosphate] and N,N'-carbonyldiimidazole. In the scope of this invention preferred amide bond linking reagents are uronium salts and, particularly, carbodiimides, preferably, 1-ethyl-3-(3-dimethylaminopropyl)carbodiimide hydrochloride.

Said reactions are carried out under conditions known to the person skilled in the art or as described exemplarily in the following examples.

As shown in the next step, compounds of the formula IV, in which R1, R2, R3, R4, R41, R5, R51 and R8 have the meanings indicated above, can be obtained by cyclocondensation of corresponding compounds of the formula VI. Said cyclocondensation reaction is carried out in a manner habitual per se to the person skilled in the art or as described by way of example in the following examples, according to Bischler-Napieralski (e.g. as described in J. Chem. Soc., 1956, 4280-4282) in the presence of a suitable condensing or dehydrating agent, such as, for example, polyphosphoric acid, phosphorus pentachloride, phosphorus pentoxide or phosphorus oxychloride, in a suitable inert solvent, e.g. in a chlorinated hydrocarbon such as chloroform, or in a cyclic hydrocarbon such as toluene or xylene, or another inert solvent such as acetonitrile, or without further solvent using an excess of condensing agent, at reduced temperature, or at room temperature, or at elevated temperature or at the boiling temperature of the solvent or condensing agent used.

Compounds of formula IV are converted either with suitable aldehydes and compounds of formula III, in which R6 is 1-6C-alkyl or 1-4C-alkyl substituted by 1-4C-alkoxycarbonyl, or with suitable nitroalkenes, optionally in an one pot synthesis and suitably in the presence of an inorganic or organic base (in particular a cyclic amine, e.g. piperidine) into corresponding compounds which comprise a structure-element of the formula X, Xa or Xb. Said conversion can be carried out as known to the skilled person or as described in the following examples or analogously or similarly thereto.

Said suitable aldehydes, nitroalkenes and compounds of formulae VIII, VII and III are commercially available or can be obtained in a manner known to the skilled person from his/her expert knowledge and/or from literature. Suitable nitroalkenes are known or are accessible by reaction of suitable aldehydes with nitro compounds of formula III in the presence of a suitable organic or inorganic base in a manner customary per se to the skilled person.

Compounds obtained can be converted into further compounds, which comprise a structure-element of the formula X, Xa or Xb, by methods known to one of ordinary skill in the art. More specifically, for example, from compounds, in which
a.) R61 are an ester group, the corresponding acids can be obtained by acidic or, particularly, alkaline hydrolysis;
b.) R6 is 1-4C-alkyl, particularly methyl, the corresponding halogenated, preferably chlorinated, groups can be obtained by halogenation reaction, particularly by reaction with a chlorination reagent such as sulfuryl chloride, thionyl chloride or N-chlorosuccinimide;
c.) R6 is 1-4C-alkyl substituted by halogen, the corresponding derivatized 1-4C-alkyl radicals substituted by 1-4C-alkoxy, hydroxyl, halogen or -N(R611)R612 can be obtained by nucleophilic substitution reactions with suitable nucleophiles;
d.) R6 is 1-4C-alkyl substituted by hydroxyl, the corresponding derivatized 1-4C-alkyl radicals substituted by 1-4C-alkoxycarbonyl can be obtained by oxidation and esterification reactions under suitable conditions;
e.) R6 is methyl, the corresponding oxidized forms thereof (e.g. the hydroxymethyl or formyl radicals) can be obtained stepwise or directly by selective oxidation reactions (e.g. with the aid of manganese dioxide to obtain the formyl radicals);
f.) R6 is formyl, the corresponding aminated compounds can be obtained by reductive amination reaction;
g.) R6 is hydroxymethyl, the corresponding fluorine compounds can be obtained by fluorination reaction;
h.) R6 is methyl, the corresponding amino compounds can be obtained by nitration reaction and subsequential reduction of the nitro compounds obtained.

The methods mentioned under a.) to h.) are expediently carried out analogously to the methods known to the person skilled in the art or as described by way of example in the following examples.

It is moreover known to the person skilled in the art that if there are a number of reactive centers on a starting or intermediate compound it may be necessary to block one or more reactive centers temporarily by protective groups in order to allow a reaction to proceed specifically at the desired reaction center. A detailed description for the use of a large number of proven protective groups is found, for example, in "Protective Groups in Organic Synthesis" by T. Greene and P. Wuts (John Wiley & Sons, Inc. 1999, 3rd Ed.) or in "Protecting Groups (Thieme Foundations Organic Chemistry Series N Group" by P. Kocienski (Thieme Medical Publishers, 2000).

The isolation and purification of the compounds according to the invention, i.e. the compounds with the structure-element of the formula X, Xa or Xb, is carried out in a manner known per se, e.g. by distilling off the solvent in vacuo and recrystallizing the resulting residue from a suitable solvent or subjecting it to one of the customary purification methods, such as, for example, column chromatography on suitable support material.

The compounds according to the present invention include suitable salt forms. Suitable salts for compounds according to the invention - depending on substitution - are all acid addition salts or all salts with bases. Particular mention may be made of the pharmacologically tolerable inorganic and organic acids and bases customarily used in pharmacy. Those suitable are, on the one hand, water-insoluble and, particularly, water-soluble acid addition salts with acids such as, for example, hydrochloric acid, hydrobromic acid, phosphoric acid, nitric acid, sulphuric acid, acetic acid, citric acid, D-gluconic acid, benzoic acid, 2-(4-hydroxybenzoyl)benzoic acid, butyric acid, sulphosalicylic acid, maleic acid, lauric acid, malic acid, fumaric acid, succinic acid, oxalic acid, tartaric acid, embonic acid, stearic acid, toluenesulphonic acid, methanesulphonic acid or 3-hydroxy-2-naphthoic acid, the acids being employed in salt preparation - depending on whether a mono- or polybasic acid is concerned and depending on which salt is desired - in an equimolar quantitative ratio or one differing therefrom.

On the other hand, salts with bases are - depending on substitution - also suitable. As examples of salts with bases are mentioned the lithium, sodium, potassium, calcium, aluminium, magnesium, titanium, ammonium, meglumine or guanidinium salts, here, too, the bases being employed in salt preparation in an equimolar quantitative ratio or one differing therefrom.

Pharmacologically intolerable salts, which can be obtained, for example, as process products during the preparation of the compounds according to the invention on an industrial scale, are converted into pharmacologically tolerable salts by processes known to the person skilled in the art.

Salts are obtained by dissolving the free compound in a suitable solvent (e.g. a ketone, such as acetone, methyl ethyl ketone or methyl isobutyl ketone, an ether, such as diethyl ether, tetrahydrofuran or dioxane, a chlorinated hydrocarbon, such as methylene chloride or chloroform, or a low molecular weight aliphatic alcohol such as ethanol or isopropanol) which contains the desired acid or base, or to which the desired acid or base is then added. The salts are obtained by filtering, reprecipitating, precipitating with a nonsolvent for the addition salt or by evaporating the solvent. Salts obtained can be converted by alkalization or by acidification into the free compounds, which in turn can be converted into salts. In this way, pharmacologically intolerable salts can be converted into pharmacologically tolerable salts.

According to expert's knowledge the compounds of the invention as well as their salts may contain, e.g. when isolated in crystalline form, varying amounts of solvents. Included within the scope of the invention are therefore all solvates and in particular all hydrates of the compounds according to the invention as well as all solvates and in particular all hydrates of the salts of the compounds according to the invention.

Depending on substitution the compounds according to the present invention can be chiral compounds having, for example, chiral centers and/or chiral axes due to hindered rotation about single bonds. The invention therefore includes all conceivable pure diastereomers and pure enantiomers and mixtures thereof in any mixing ratio including the racemates. The diastereomer mixtures can be separated into the individual isomers by chromatographic processes. The enantiomers can be separated in a known manner (e.g. by chromatographic processes on chiral phases or by resolution).

The person skilled in the art knows on the basis of his/her knowledge and on the basis of those synthesis routes, which are shown and described within the description of this invention, how to find other possible synthesis routes compounds according to the invention.

The following examples serve to illustrate the invention in greater detail. Likewise, further compounds which comprise a structure-element of the formula X, Xa or Xb, whose preparation is not explicitly described, can also be prepared in an analogous manner or in a manner familiar per se to the person skilled in the art using customary process techniques.

In the examples, m.p. stands for melting point, h for hour(s), min for minutes, conc. for concentrated, satd. for saturated, MS for mass spectrum, M for molecular ion.

Unless otherwise noted, if the exemplary compounds mentioned expressis verbis herein contain a chirality center, they are desribed illustratively as racemic mixtures herein.

The compounds mentioned in the examples as well as their salts and stereoisomers are a particular interesting subject of the invention.

### Examples

### Final products

1. 2-(4-Hydroxy-3,5-dimethyl-phenyl)-8,9-dimethoxy-3,5,5-trimethyl-5,6-dihydro-pyrrolo[2,1-a]isoquinoline-1-carboxylic acid ethyl ester
   Analogously to a procedure described by Meyer in Liebigs Ann. Chem. 1981, 9, 1534-1544, (6,7-dimethoxy-3,3-dimethyl-3,4-dihydro-2H-isoquinolin-1-ylidene)-acetic acid ethyl ester (compound A7) is reacted with nitro ethane and 4-hydroxy-3,5-dimethyl benzaldehyde to afford the title compound. MS (M+H) = 464.1; m.p. = 210 - 213 °C
   The following examples (Examples 2-20) can be prepared in analogy to example 1 using the appropriate starting compound selected from the group consisting of the compounds A1 to A9. All aldehydes used are commercially available or can be prepared in analogy to published procedures. If nitro propane or 4-nitro butyric acid methyl ester is used instead of nitroethane, 3-ethyl-5,6-dihydro-pyrrolo[2,1-a]isoquinolines and 3-methoxycarbonylethyl-5,6-dihydro-pyrrolo[2,1-a]isoquinolines (e.g. 3-(8,9-dimethoxy-5,6-dihydro-pyrrolo[2,1-a]isoquinolin-3-yl)propionic methyl esters), respectively are obtained.
2. 8,9-Dimethoxy-3,5,5-trimethyl-2-(3,4,5-trimethoxy-phenyl)-5,6-dihydro-pyrrolo[2,1-a]isoquinoline-1-carboxylic acid ethyl ester
   MS (M+H) = 510.4; m.p. = 52 - 56 °C
3. 2-[3-(4-Chloro-phenoxy)-phenyl]-8,9-dimethoxy-3,5,5-trimethyl-5,6-dihydro-pyrrolo[2,1-a]isoquinoline-1-carboxylic acid ethyl ester
   MS (M+H) = 546.2; m.p. = 61 - 64 °C
4. 2-(3-Dimethylamino-phenyl)-8,9-dimethoxy-3,5,5-timethyl-5,6-dihydro-pyrrolo[2,1-a]isoquinoline-1-carboxylic acid ethyl ester
   MS (M+H) = 463.1; m.p. =101 - 102 °C
5. (5RS)- (4-Hydroxy-3,5-dimethyl-phenyl)-8,9-dimethoxy-3,5-dimethyl-5,6-dihydro-pyrrolo[2,1-a]isoquinoline-1-carboxylic acid ethyl ester
   MS (M+H) = 450.2; m.p. =158 -161 °C
6. (5RS)-5-Ethyl-2-(4-hydroxy-3,5-dimethyl-phenyl)-8,9-dimethoxy-3-methyl-5,6-dihydro-pyrrolo[2,1-a]isoquinoline-1-carboxylic acid ethyl ester
   MS (M+H) = 464.1; m.p. =164 - 166 °C
7. (5RS)-2-Chlorophenyl-5-ethyl-8,9-dimethoxy-3-methyl-5,6-dihydro-pyrrolo[2,1-a]isoquinoline-1-carboxylic acid ethyl ester
   MS (M+H) = 454.2; m.p. =121 -124 °C
8. (4aRS,8aRS)-cis-2-(4-hydroxy-3,5-dimethyl-phenyl)-10,11-dimethoxy-3-methyl-4a,5,6,7,8,8a-hexahydro-pyrrolo[2,1-f]phenanthridine-1-carboxylic acid ethyl ester
   MS (M+H) = 490.2; m.p. =186 -192 °C
9. (5RS)-3-Ethyl-2-(4-hydroxy-3,5-dimethyl-phenyl)-8,9-dimethoxy-5-methyl-5,6-dihydro-pyrrolo[2,1-a]isoquinoline-1-carboxylic acid ethyl ester
   MS (M+H) = 464.1; m.p. = 188 -190 °C
10. (5RS)-8,9-Dimethoxy-3,5-dimethyl-2-(3,4,5-trimethoxy-phenyl)-5,6-dihydro-pyrrolo[2,1-a]isoquinoline-1-carboxylic acid ethyl ester
   MS (M+H) = 496.0; m.p. = 116 - 118 °C
11. (5RS)-8,9-Dimethoxy-3,5-dimethyl-2-naphthalen-1-yl-5,6-dihydro-pyrrolo[2,1-a]isoquinoline-1-carboxylic acid ethyl ester
   MS (M+H) = 456.1; m.p. = 184 °C
12. (4aRS,8aRS)-cis-10,11-Dimethoxy-3-methyl-2-naphthalen-1-yl-4a,5,6,7,8,8a-hexahydro-pyrrolo[2,1-f]phenanthridine-1-carboxylic acid ethyl ester
   MS (M+H) = 496.1; m.p. = 189 -191 °C
13. (4aRS,8aRS)-cis-10,11-Dimethoxy-3-methyl-2-quinolin-4-yl-4a,5,6,7,8,8a-hexahydro-pyrrolo[2,1-f]phenanthridine-1-carboxylic acid ethyl ester
   MS (M+H) = 497.3; m.p. =153 -157 °C
14. (4aR,8aR)-10,11-Dimethoxy-3-methyl-2-quinolin-4-yl-4a,5,6,7,8,8a-hexahydro-pyrrolo[2,1-f]phenanthridine-1-carboxylic acid ethyl ester
   MS (M+H) = 497.3; oil
15. (4aR,8aR)-10,11-Dimethoxy-3-methyl-2-naphthalen-1-yl-4a,5,6,7,8,8a-hexahydro-pyrrolo[2,1-f]phenanthridine-1-carboxylic acid ethyl ester
   MS (M+H) = 496.1; m.p. = 212 - 216 °C
16. (4aR,8aR)-2-(4-Hydroxy-3,5-dimethyl-phenyl)-10,11-dimethoxy-3-methyl-4a,5,6,7,8,8a-hexahydro-pyrrolo[2,1-f]phenanthridine-1-carboxylic acid ethyl ester
   MS (M+H) = 490.2; m.p. = 203 - 206 °C
17. (5RS)-5-Ethyl-8,9-dimethoxy-3-methyl-2-naphthalen-1-yl-5,6-dihydro-pyrrolo[2,1-a]isoquinoline-1-carboxylic acid ethyl ester
   MS (M+H) = 470.1; oil
18. (5RS)-2-(4-Hydroxy-3,5-dimethyl-phenyl)-7,8,9-trimethoxy-3,5-dimethyl-5,6-dihydro-pyrrolo[2,1-a]isoquinoline-1-carboxylic acid ethyl ester
   MS (M+H) = 480.0; m.p. = 144 °C
19. 2-(4-Hydroxy-3,5-dimethyl-phenyl)-8,9-dimethoxy-5,6-dihydro-pyrrolo[2,1-a]isoquinoline-1,5-dicarboxylic acid 1-ethyl 5-methyl ester
   MS (M+H) = 494.1; m.p. = 92 - 97 °C
20. (5RS)-8,9-Dimethoxy-3-(2-methoxycarbonyl-ethyl)-5-methyl-2-naphthalen-1-yl-5,6-dihydro-pyrrolo[2,1-a]isoquinoline-1-carboxylic acid ethyl ester
   MS (M+H) = 528.1; m.p. = 56 - 59 °C
21. 2-(4-Hydroxy-3,5-dimethyl-phenyl)-8,9-dimethoxy-3-methyl-5,6-dihydro-pyrrolo[2,1-a]isoquinoline-1-carbonitrile
   Analogously to the procedure described for Example 1, (6,7-dimethoxy-3,4-dihydro-2H-isoquinolin-1-ylidene)-acetonitrile (compound A8) is reacted with nitro ethane and 4-hydroxy-3,5-dimethyl benzaldehyde to afford 2-(4-hydroxy-3,5-dimethyl-phenyl)-8,9-dimethoxy-3-methyl-5,6-dihydro-pyrollo[2,1-a]isoquinoline-1-carbonitrile as a colorless solid of m.p. 285 - 287 °C. The mass spectrum shows the molecular peak M+H at 388.5 Da.
   The following examples (Nos. 22-28) can be prepared in analogy to example 21 using the appropriate starting compound A8 or A9. All aldehydes used are commercially available or can be prepared in analogy to published procedures. If nitro propane or 4-nitro butyric acid methyl ester is used instead of nitroethane, 3-ethyl-5,6-dihydro-pyrrolo[2,1-a]isoquinolines and 3-methoxycarbonylethyl-5,6-dihydro-pyrrolo[2,1-a]isoquinolines, respectively are obtained.
22. 8,9-Dimethoxy-3-methyl-2-naphthalen-1-yl-5,6-dihydro-pyrrolo[2,1-a]isoquinoline-1-carbonitrile
   MS (M+H) = 395.2; m.p. = 226 - 229 °C
23. 8,9-Dimethoxy-3-methyl-2-quinolin-4-yl-5,6-dihydro-pyrrolo[2,1-a]isoquinoline-1-carbonitrile
   MS (M+H) = 396.3; m.p. = 239 -243 °C
24. 2-(1H-Indol-3-yl)-8,9-dimethoxy-3-methyl-5,6-dihydro-pyrrolo[2,1-a]isoquinoline-1-carbonitrile
   MS (M+H) = 384.3; m.p. = 304 - 307 °C
25. 2-(3,5-Di-tert-butyl-4-hydroxy-phenyl)-8,9-dimethoxy-3-methyl-5,6-dihydro-pyrrolo[2,1-a]isoquinoline-1-carbonitrile
   MS (M+H) = 473.1; m.p. = 250 - 252 °C
26. 8,9-Dimethoxy-3,5-dimethyl-2-pyridin-4-yl-5,6-dihydro-pyrrolo[2,1-a]isoquinoline-1-carbonitrile
   MS (M+H) = 360.3; m.p. = 253 - 254 °C
27. 3-[1-Cyano-2-(4-hydroxy-3,5-dimethyl)-8,9-dimethoxy-5-methyl-5,6-dihydro-pyrrolo[2,1-a]isoquinolin3-yl]-propionic acid methyl ester
   MS (M+H) = 475.2; m.p. = 208 - 209 °C
28. 2-(4-Hydroxy-3,5-dimethyl-phenyl)-8,9-dimethoxy-3,5-dimethyl-5,6-dihydro-pyrrolo[2,1-a]isoquinoline-1-carbonitrile
   MS (M+H) = 403.2; m.p. = 268 - 270 °C
   The following examples (Nos. 29-59) can be prepared in analogy to example 21 using the appropriate starting compound, which can be prepared in an art-known manner, or analogously or similarly as described for A8 or A9. All aldehydes used are commercially available or can be prepared in analogy to published procedures. If nitro propane or 4-nitro butyric acid methyl ester is used instead of nitroethane, 3-ethyl-5,6-dihydro-pyrrolo[2,1-a]isoquinolines and 3-methoxycarbonylethyl-5,6-dihydro-pyrrolo[2,1-a]isoquinofines, respectively are obtained.
29. 3-(1-Cyano-8,9-dimethoxy-2-pyridin-4-yl-5,6-dihydro-pyrrolo[2,1-a]isoquinolin-3-yl)-propionic acid methyl ester
   MS (M+H) = 417.9; m.p. = 191 - 193 °C
30. 7-Fluoro-2-(4-hydroxy-3,5-dimethyl-phenyl)-8,9-dimethoxy-3,5-dimethyl-5,6-dihydro-pyrrolo[2,1-a]isoquinoline-1-carbonitrile
   MS (M+H) = 421.2; m.p. = 166 -168 °C
31. 3-(1-Cyano-8,9-dimethoxy-2-quinolin-4-yl-5,6-dihydro-pyrrolo[2,1-a]isoquinolin-3-yl)-propionic acid methyl ester
   MS (M+H) = 467.9; m.p. = 232 - 234 °C
32. 3-[1-Cyano-2-(4-hydroxy-3,5-dimethyl-phenyl)-8,9-dimethoxy-5,6-dihydro-pyrrolo[2,1-a]isoquinolin-3-yl]-propionic acid methyl ester
   MS (M+H) = 461.0; m.p. = 217 - 219 °C
33. 8,9-Dimethoxy-2-(4-methoxy-3,5-dimethyl-phenyl)-3,5-dimethyl-5,6-dihydro-pyrrolo[2,1-a]isoquinoline-1-carbonitrite
   MS (M+H) = 417.3;
34. 2-(1 H-Indol-5-yl)-8,9-dimethoxy-3-methyl-5,6-dihydro-pyrrolo[2,1-a]isoquinoline-1-carbonitrile
   MS (M+H) = 384.3;
35. 8,9-Dimethoxy-2-(4-methoxy-3,5-dimethyl-phenyl)-3-methyl-5,6-dihydro-pyrrolo[2,1-a]isoquinoline-1-carbonitrile
   MS (M+H) = 403.3;
36. 2-(1-Benzyl-2,3-dihydro-1H-indol-5-yl)-8,9-dimethoxy-3-methyl-5,6-dihydro-pyrrolo[2,1-a]isoquinoline-1-carbonitrile
   MS (M+H) = 476.1;
37. 8,9-Dimethoxy-3,5-dimethyl-2-[1-(toluene-4-sulfonyl)-1H-pyrrol-3-yl]-5,6-dihydro-pyrrolo[2,1-a]isoquinoline-1-carbonitrile
   MS (M+H) = 502.1;
38. 8,9-Dimethoxy-3,5-dimethyl-2-[1-(toluene-4-sulfonyl)-1H-indol-3-yl]-5,6-dihydro-pyrrolo[2,1-a]isoquinoline-1-carbonitrile
   MS (M+H) = 569.0;
39. 2-(1-Benzenesulfonyl-1H-indol-3-yl)-8,9-dimethoxy-3,5-dimethyl-5,6-dihydro-pyrrolo[2,1-a]isoquinoline-1-carbonitrile
   MS (M+H) = 537.7;
40. 2-(1-Methanesulfonyl-1H-indol-3-yl)-8,9-dimethoxy-3,5-dimethyl-5,6-dihydro-pyrrolo[2,1-a]isoquinoline-1-carbonitrile
   MS (M+H) = 475.8; m.p. = 219 - 221 °C
41. 8,9-Dimethoxy-3,5-dimethyl-2-(1-oxy-pyridin-4-yl)-5,6-dihydro-pyrrolo[2,1-a]isoquinoline-1-carbonitrile
   MS (M+H) = 375.8; m.p. = 279 - 282 °C
42. 7-Fluoro-8,9-dimethoxy-3,5-dimethyl-2-[1-(toluene-4-sulfonyl)-1H-indol-3-yl]-5,6-dihydro-pyrrolo[2,1-a]isoquinoline-1-carbonitrile
   MS (M+H) = 587.0;
43. 2-(2,3-Dihydro-1H-indol-5-yl)-8,9-dimethoxy-3-methyl-5,6-dihydro-pyrrolo[2,1-a]isoquinoline-1-carbonitrile
   MS (M+H) = 386.3;
44. 2-(4-Hydroxy-3,5-dimethyl-phenyl)-8,9-dimethoxy-5-methyl-3-morpholin-4-ylmethyl-5,6-dihydro-pyrrolo[2,1-a]isoquinoline-1-carbonitrile
   m.p. = 228 - 230 °C
45. 8,9-Dimethoxy-3,5-dimethyl-2-(2-methyl-pyridin-4-yl)-5,6-dihydro-pyrrolo[2,1-a]isoquinoline-1-carbonitrile
   MS (M+H) = 374.2; m.p. = 187 - 189 °C
46. 8,9-Dimethoxy-3,5-dimethyl-2-(4-nitro-phenyl)-5,6-dihydro-pyrrolo[2,1-a]isoquinoline-1-carbonitrile
   MS (M+H) = 403.7; m.p. = 206 - 207 °C
47. 4-(1-Cyano-8,9-dimethoxy-3,5-dimethyl-5,6-dihydro-pyrrolo[2,1-a]isoquinolin-2-yl)-benzoic acid
   MS (M+H) = 402.7; m.p. = 287 - 289 °C
48. 2-(4-Amino-phenyl)-8,9-dimethoxy-3,5-dimethyl-5,6-dihydro-pyrrolo[2,1-a]isoquinoline-1-carbonitrile
   MS (M+H) = 374.1; m.p. = 237 - 239 °C
49. 8,9-Dimethoxy-3,5-dimethyl-2-(3-methyl-pyridin-4-yl)-5,6-dihydro-pyrrolo[2,1-a]isoquinoline-1-carbonitrile
   MS (M+H) = 374.5; m.p. = 232 - 233 °C
50. 4-(1-Cyano-8-ethoxy-9-methoxy-3,5-dimethyl-5,6-dihydro-pyrrolo[2,1-a]isoquinolin-2-yl)-benzoic acid
   MS (M+H) = 417.2; m.p. = 274 - 277 °C
51. 2-(4-Hydroxy-2-methyl-phenyl)-8,9-dimethoxy-3,5-dimethyl-5,6-dihydro-pyrrolo[2,1-a]isoquinoline-1-carbonitrile
   MS (M+H) = 389.1; m.p. = 228 - 230 °C
52. 4-(1-Cyano-8,9-dimethoxy-3,5-dimethyl-5,6-dihydro-pyrrolo[2,1-a]isoquinolin-2-yl)-benzamide
   m.p. = 228 - 230 °C
53. 8-Ethoxy-2-(4-hydroxy-3,5-dimethyl-phenyl)-9-methoxy-3,5-dimethyl-5,6-dihydro-pyrrolo[2,1-a]isoquinoline-1-carbonitrile
   MS (M+H) = 417.2; m.p. = 232 - 234 °C
54. 3-(1-Cyano-8,9-dimethoxy-3,5-dimethyl-5,6-dihydro-pyrrolo[2,1-a]isoquinolin-2-yl)-indole-1-sulfonic acid dimethylamide
   MS (M+H) = 505.2; m.p. = 236 - 237 °C
55. 8,9-Dimethoxy-3,5-dimethyl-2-(2-methyl-1-oxy-pyridin-4-yl)-5,6-dihydro-pyrrolo[2,1-a]isoquinoline-1-carbonitrile
   MS (M+H) = 390.1; m.p. = 265 - 268 °C
56. 8,9-Dimethoxy-3,5-dimethyl-2-[1-(morpholine-4-sulfonyl)-1H-indol-3-yl]-5,6-dihydro-pyrrolo[2,1-a]isoquinoline-1-carbonitrile
   MS (M+H) = 574.1; m.p. = 210 - 212 °C
57. 8,9-Dimethoxy-3,5-dimethyl-2-[4-(2H-tetrazol-5-yl)-phenyl]-5,6-dihydro-pyrrolo[2,1-a]isoquinoline-1-carbonitrile
   MS (M+H) = 427.2; m.p. = 204 - 207 °C
58. Morpholine-4-sulfonic acid [4-(1-cyano-8,9-dimethoxy-3,5-dimethyl-5,6-dihydro-pyrrolo[2,1 a]isoquinolin-2-yl)-phenyl]-amide
   MS (M+H) = 523.1; m.p. = 223 - 225 °C
59. N-[4-(1-Cyano-8,9-dimethoxy-3,5-dimethyl-5,6-dihydro-pyrrolo[2,1-a]isoquinolin-2-yl)-phenyl]-methanesulfonamide
   MS (M+H) = 452.1; m.p. = 257 - 259 °C
   The following examples (Examples 60-67) can be prepared in analogy to example 1 using the appropriate starting compound, which can be prepared in an art-known manner, or analogously or similarly as described for compounds A1 to A9. All aldehydes used are commercially available or can be prepared in analogy to published procedures. If nitro propane or 4-nitro butyric acid methyl ester is used instead of nitroethane, 3-ethyl-5,6-dihydro-pyrrolo[2,1-a]isoquinolines and 3-methoxycarbonylethyl-5,6-dihydro-pyrrolo[2,1-a]isoquinolines, respectively are obtained.
60. 5-Ethyl-2-(2-fluoro-3,4-dimethoxy-phenyl)-8,9-dimethoxy-3-methyl-5,6-dihydro-pyrrolo[2,1-a]isoquinoline-1-carboxylic acid ethyl ester
   MS (M+H) = 498.1;
61. 7-Chloro-8,9-dimethoxy-3,5-dimethyl-2-pyridin-4-yl-5,6-dihydro-pyrrolo[2,1-a]isoquinoline-1-carboxylic acid ethyl ester
   MS (M+H) = 441.3;
62. 7-Chloro-2-(4-hydroxy-3,5-dimethyl-phenyl)-8,9-dimethoxy-3,5-dimethyl-5,6-dihydro-pyrrolo[2,1-a]isoquinoline-1-carboxylic acid ethyl ester
   MS (M+H) = 484.0;
63. 7,8,9-Trimethoxy-3,5-dimethyl-2-pyridin-4-yl-5,6-dihydro-pyrrolo[2,1-a]isoquinoline-1-carboxylic acid ethyl ester
   MS (M+H) = 437.3;
64. 8,9-Dimethoxy-3-(2-methoxycarbonyl-ethyl)-5-methyl-2-quinolin-4-yl-5,6-dihydro-pyrrolo[2,1-a]isoquinoline-1-carboxylic acid ethyl ester
   MS (M+H) = 529.3;
65. 2-(4-Hydroxy-3,5-dimethyl-phenyl)-8,9-dimethoxy-3,5-dimethyl-5,6-dihydro-pyrrolo[2,1-a]isoquinoline-1-carboxylic acid methyl ester
   MS (M+H) = 435.9; m.p. = 177 - 179 °C
66. 8,9-Dimethoxy-3,5-dimethyl-2-[1-(toluene-4-sulfonyl)-1H-indol-3-yl]-5,6-dihydro-pyrrolo[2,1-a]isoquinoline-1-carboxylic acid methyl ester
   MS (M+H) = 584.9; m.p. = 177 - 179 °C
67. 5-Cyano-2-(4-hydroxy-3,5-dimethyl-phenyl)-8,9-dimethoxy-3-methyl-5,6-dihydro-pyrrolo[2,1-a]isoquinoline-1-carboxylic acid ethyl ester
   MS (M+H) = 461.0;
68. 4-(8,9-Dimethoxy-1,3-dimethyl-5,6-dihydro-pyrrolo[2,1-a]isoquinolin-2-yl)-2,6-dimethyl-phenol
   MS (M+H) = 377.9; m.p. = 183 - 185 °C
   The title compound can be obtained via an analogous synthesis route as described for the Examples herein.
69. 8,9-Dimethoxy-3-(2-methoxycarbonyl-ethyl)-5-methyl-2-quinolin-4-yl-5,6-dihydro-pyrrolo[2,1-a]isoquinoline-1-carboxylic acid ethyl ester
   MS (M+H) = 407.9; m.p. = 176 - 177 °C
   The title compound can be obtained from the corresponding ester compound, which is accessible analogously as described in Example 1 herein, by art-known saponification reaction.

Examplary compounds of formula Ic mentioned as Examples 70-139 in Tables A1 to A4 can be prepared analogously or similarly to the described examples.

Examplary compounds of formulae Id or Ie can be also prepared analogously or similarly to the described examples.

### Starting compounds

- A1: (3RS)-(6,7-Dimethoxy-3-methyl-3,4-dihydro-2H-isoquinolin-1-ylidene)-acetic acid ethyl ester
The title compound can be obtained by a Bischler-Napieralski reaction (Ber. 1893, 26, 1903) using N-{2-[4-methoxy-3-(2-methoxy-ethoxy)-phenyl]-ethyl}-malonamic acid ethyl ester (compound B1) as the starting material.

The following 3,4-Dihydro-1(2H)-isoquinolinylidene-derivatives A2 to A9 and also further relevant, non-explicitly described similar compounds can be prepared according to an analogous procedure using the appropriate starting compound B2 to B8, or an appropriate analogous compound:
- A2: (3RS)-(3-Ethyl-6,7-dimethoxy-3,4-dihydro-2H-isoquinolin-1-ylidene)-acetic acid ethyl ester
- A3: ((4aR,10bR)-8,9-Dimethoxy-1,3,4,4a,5,10b-hexyhydro-2H-phenanthridin-8-ylidene)-acetic acid ethyl ester.
- A4: ((4aRS,10bRS)-cis-8,9-Dimethoxy-1,3,4,4a,5,10b-hexyhydro-2H-phenanthridin-6-ylidene)-acetic acid ethyl ester
- A5: 1-Ethoxycarbonylmethylene-6,7-dimethoxy-1,2,3,4-tetrahydro-isoquinolie-3-carboxylic acid methyl ester
- A6: (3RS)-(5,6,7-Trimethoxy-3-methyl-3,4-dihydro-2H-isoquinolin-1-ylidene)-acetic acid ethyl ester
- A7: (6,7-Dimethoxy-3,3-dimethyl-3,4-dihydro-2H-isoquinolin-1-ylidene)-acetic acid ethyl ester The compound A19 is commercially available.
- A8: (6,7-Dimethoxy-3,4-dihydro-2H-isoquinolin-1-ylidene)-acetonitrile The compound A8 can be prepared analogously to the above-described synthesis of compound A1 using the starting compound B7.
- A9: (6,7-Dimethoxy-3-methyl-3,4-dihydro-2H-isoquinolin-1-ylidene)-acetonitrile The compound A9 can be prepared analogously to the above-described synthesis of compound A1 using the starting compound B8.

- B1: N-[(RS)-2-(3,4-Dimethoxy-phenyl)-1-methyl-ethyl]-malonamic acid ethyl ester
The title compound can be prepared by a reaction of (RS)-2-(3,4-Dimethoxy-phenyl)-1-methyl-ethylamine (compound C1) with ethyl maloyl chloride in analogy to procedures in the literature (e.g. Benovsky et al., Tetrahedron Lett. 1997, 38, 8475-8478).

The following amides B2 to B8 can be synthesized according an analogous procedure:
- B2: N-[(RS)-1-(3,4-Dimethoxy-benzyl)-propyl]-malonamic acid ethyl ester
- B3: N-[(1R,2R)-2-(3,4-Dimethoxy-phenyl)-cyclohexyl]-malonamic acid ethyl ester
- B4: N-[(1RS,2RS)-cis-2-(3,4-Dimethoxy-phenyl)-cyclohexyl]-malonamic acid ethyl ester
- B5: 3-(3,4-Dimethoxy-phenyl)-2-(2-ethoxycarbonyl-ethanoylamino)-propionic acid methyl ester
- B6: N-[(RS)-1-Methyl-2-(2,3,4-trimethoxy-phenyl)-ethyl]malonamic acid ethyl ester

- B7: 2-Cyano-N-[2-(3,4-dimethoxy-phenyl)-ethyl]-acetamide

A solution of 10.0 g (55.1 mmol) of 2-(3,4-dimethoxy-phenyl)-ethylamine and 9.36 g (82.7 mmol) of ethyl cyano acetate is stirred at 100 °C for 15 h. The mixture is cooled to room temperature. The precipitate is filtered off and recrystallized from ethanol. 9.44 g (38.0 mmol, 60 %) of 2-cyano-N-[2-(3,4-dimethoxy-phenyl)-ethyl]-acetamide are obtained as a beige solid.
MS (M+H) = 249.0, m.p. = 113-115 °C.
- B8: 2-Cyano-N-[2-(3,4-dimethoxy-phenyl)-1-methyl-ethyl]-acetamide
Compound B8 can be prepared analogously to the synthesis of compound B7.

The appropriate starting compounds for the preparation of the compounds B1 to B8 are commercially available, or can be prepared as described below in the synthesis of the compound C1 or analogously or similarly thereto, or can be obtained in analogy to published procedures, e.g. the substituted 2-phenethyl amines can be prepared starting from the corresponding benzaldehydes (see also Shepard et al., J. Org. Chem. 1952, 17, 568).
- C1: (RS)-2-(3,4-Dimethoxy-phenyl)-1-methyl-ethylamine
The title compound can be prepared by a sequence described by Shepard et al. in J. Org. Chem. 1952, 17, 568.

### Commercial utility

### Commercial applicability

Intracellular levels of the second messengers cAMP and cGMP are regulated by both their rates of synthesis by cyclases and their hydrolysis by phosphodiesterases. Of the 11 phosphodiesterase (PDE) isoenzymes which are presently known, PDE10 was described for the first time in 1999 (Soderling SH, Bayuga SJ, Beavo JA. Isolation and characterization of a dual-substrate phosphodiesterase gene family: PDE10A. Proc Natl Acad Sci U S A. 1999 Jun 8;96(12):7071-6; Fujishige K, Kotera J, Michibata H, Yuasa K, Takebayashi S, Okumura K, Omori K. Cloning and characterization of a novel human phosphodiesterase that hydrolyzes both cAMP and cGMP (PDE10A). J Biol Chem. 1999 Jun 25;274(26):18438-45; Loughney K, Snyder PB, Uher L, Rosman GJ, Ferguson K, Florio VA. Isolation and characterization of PDE10A, a novel human 3', 5'-cyclic nucleotide phosphodiesterase. Gene. 1999 Jun 24;234(1):109-17). The first gene of this new PDE subfamily was designated PDE10A and the first splice variant was described as PDE10A1, according to the current nomenclature. Due to alternative splicing, other splice variants of PDE10A exist and have been described in the subsequent years (Kotera J, Fujishige K, Yuasa K, Omori K. Characterization and phosphorylation of PDE10A2, a novel alternative splice variant of human phosphodiesterase that hydrolyzes cAMP and cGMP. Biochem Biophys Res Commun. 1999 Aug 11;261(3):551-7; Fujishige K, Kotera J, Omori K. Striatum-and testis-specific phosphodiesterase PDE10A isolation and characterization of a rat PDE10A. Eur J Biochem. 1999 Dec;266(3):1118-27; Fujishige K, Kotera J, Yuasa K, Omori K. The human phosphodiesterase PDE10A gene genomic organization and evolutionary relatedness with other PDEs containing GAF domains. Eur J Biochem. 2000 Oct;267(19):5943-51). PDE10A has been described as a cyclic nucleotide phosphodiesterase exhibiting properties of a cAMP PDE and a cAMP-inhibited cGMP PDE.

Individual representatives of the PDE10 isoenzyme are characterized by being particularly prominently expressed in specific areas of the brain (striatum, putamen, caudate nucleus, cerebellum, thalamus), in testis, in the thyroid gland, in the pituitary gland, in kidney and in placenta. Increased expression levels in a broad variety of tumor cell lines and tissues, namely of the lung, breast, pancreas, brain, prostate and ovar indicates that PDE10 may play an important role in tumor cell growth and/or survival under conditions of elevated cAMP and/or cGMP generation. Increased expression levels and activities of PDE10A have been also found in testis suggesting that PDE10A may contribute to spermatogenesis (Fujishige K et al., Eur J Biochem. 1999, 266:1118-27). Certain PDE inhibitors, namely e.g. PDE3 or PDE11A inhibitors, are known to augment glucose-induced insulin secretion and thus may be useful for treating diabetes (see e.g. WO 03/077949).

Accordingly, the compounds according to the invention, i.e. compounds containing a structure-element of the formula X, Xa or Xb, in which R1, R2, R3, R4, R41, R5, R51, R6 and R8 have the meanings given above in the special aspect A or in the subaspects thereto, have miscellaneous valuable pharmacological properties which make them commercially utilizable.
Thus, for example, the compounds according to this invention are PDE inhibitors.
Yet thus, for example, the compounds according to the invention are potent PDE10 inhibitors, some of which are apparently selective (by >100 fold) among other PDE isoenzymes, whereby these selective compounds are particularly preferred in the context of the present invention. The compounds according to the invention therefore can be employed as therapeutic agents for the treatment or prophylaxis of diseases in human and veterinary medicine.

Due to their potent and selective PDE10 inhibitory activity, the compounds according to the present invention may be, in a first facet of the present invention, of potential value in treating disorders of the central nervous system, in particular neurologic and psychiatric disorders, for example those mentioned in EP 1250923 and/or, in more particular, psychotic disorders, anxiety disorders, mood disorders or episodes, drug addiction, movement disorders or disorders comprising deficient cognition as a symptom (e.g. dementia, Parkinson's disease or Alzheimer's disease).

Furthermore, the compounds according to the present invention may be, in a second facet of the present invention, of potential value in treating certain disorders of the central nervous system, in particular neurologic and psychiatric disorders, for example those mentioned generically, specifically or exemplarily in EP 1250923, US 2003/0008806 and/or US 2003/0018047, such as, for example, anxiety or psychotic disorders, movement disorders, obsessive/compulsive disorders, drug addictions, cognition deficiency disorders, mood disorders or mood episodes, or neurodegenerative disorders.

In this context, examples of anxiety disorders, which may be treated by the compounds according to the present invention, include, panic disorder, agoraphobia, a specific phobia, social phobia, obsessive-compulsive disorder, post-traumatic stress disorder, acute stress disorder, or generalized anxiety disorder.

Examples of psychotic disorders, which may be treated by the compounds according to the present invention, include, schizophrenia (for example of the paranoid, disorganized, catatonic, undifferentiated, or residual type), schizophreniform disorder, schizoaffective disorder (for example of the delusional type or the depressive type), delusional disorder, substance-induced psychotic disorder (for example psychosis induced by alcohol, amphetamine, cannabis, cocaine, hallucinogens, inhalants, opioids, or phencyclidine), personality disorder of the paranoid type, or personality disorder of the schizoid type.

Examples of movement disorders, which may be treated by the compounds according to the present invention, include, Parkinson's disease, or restless leg syndrome.

Examples of obsessive/compulsive disorders, which may be treated by the compounds according to the present invention, include, Tourette's syndrome, or other tic disorders.

Examples of drug addictions, which may be treated by the compounds according to the present invention, include, an alcohol, amphetamine, cocaine, or opiate addiction.

Examples of cognition deficiency disorders, which may be treated by the compounds according to the present invention, include, Alzheimer's disease, multi-infarct dementia, alcoholic dementia or other drug-related dementia, dementia associated with intracranial tumors or cerebral trauma, dementia associated with Huntington's disease or Parkinson's disease, or AIDS-related dementia, delirium, amnestic disorder, post-traumatic stress disorder, mental retardation, a learning disorder, for example reading disorder, mathematics disorder, or a disorder of written expression, attention-deficit/hyperactivity disorder, or age-related cognitive decline.

Examples of mood disorders or mood episodes, which may be treated by the compounds according to the present invention, include, a major depressive episode of the mild, moderate or severe type, a manic or mixed mood episode, a hypomanic mood episode, a depressive episode with a typical features, a depressive episode with melancholic features, a depressive episode with catatonic features, a mood episode with postpartum onset, post-stroke depression, major depressive disorder, dysthymic disorder, minor depressive disorder, premenstrual dysphoric disorder, post-psychotic depressive disorder of schizophrenia, a major depressive disorder superimposed on a psychotic disorder such as delusional disorder or schizophrenia, a bipolar disorder (for example bipolar I disorder, bipolar II disorder), or cyclothymic disorder.

Examples of neurodegenerative disorders, which may be treated by the compounds according to the present invention, include, Parkinson's disease, Huntington's disease, dementia (for example Alzheimer's disease, multi-infarct dementia, AIDS-related dementia, or Fronto temperal Dementia), neurodegeneration associated with cerebral trauma, neurodegeneration associated with stroke, neurodegeneration associated with cerebral infarct, hypoglycemia-induced neurodegeneration, neurodegeneration associated with epileptic seizure, neurodegeneration associated with neurotoxin poisoning, or multi-system atrophy.

Yet in this context, the compounds according to the present invention may be of potential value for treating diseases or conditions, in which abnormal function of the basal ganglia has been implicated. Thus, abnormal function of the basal ganglia may be involved in disregulated motoric, appetitive and/or cognitive processes. Exemplary neuropsychiatric conditions, in which abnormal function of the basal ganglia has been implicated, are mentioned e.g. in EP 1250923, US 2003/0008806 and/or US 2003/0018047, such as e.g. psychosis, attention-deficit/hyperactivity disorder (ADHD) and related attentional disorders, depression, obsessive conpulsive disorders including Tourette's syndrome and other tic disorders, and substance abuse. Several neurological disorders including Parkinson's disease, restless leg syndrom and Huntington's disease can be also linked to basal ganglia dysfunction.

Still yet in this context, the compounds according to the present invention may be of potential value for improving cognition, powers of concentration, learning skills or hypermesia, in particular if the disorder is a symptom of dementia.

Yet furthermore, the compounds according to the present invention may be, in a third facet of the present invention, of potential value for regulating fertility in men, e.g. via reducing spermatogenesis and/or via reducing sperm motility.

Still yet furthermore, the compounds according to the present invention may be, in a fourth facet of the present invention, of potential value for treating diabetes, such as, for example, typ II diabetes, e.g. via augmenting glucose-induced insulin secretion.

A special interest in the compounds according to the present invention lies in their use in therapy of schizophrenia.

Another special interest in the compounds according to the present invention lies in their use in the therapy of psychotic disorders.

Another special interest in the compounds according to the present invention lies in their use in the therapy of drug addictions.

The invention further relates to a use of the claimed compounds in a composition for treating mammals, including humans, which/who are suffering from one of the abovementioned diseases and/or disorders. The use is characterized by the fact that a pharmacologically active and therapeutically effective and tolerated quantity of one or more of the compounds according to the invention is administered to the affected mammal.

The invention further relates to a use of the claimed compounds in a composition for treating mammals, in particular humans, which/who are suffering from one of the abovementioned diseases and/or disorders comprising the step of administering to said ill mammal a pharmaceutically acceptable composition according to the present invention.

The invention further relates to the compounds according to the invention for use in the treatment or prophylaxis of diseases, in particular said diseases and/or disorders.

The invention likewise relates to the use of the compounds according to the invention in the manufacture of pharmaceutical compositions which are employed for the treatment of said diseases or disorders.

The invention further relates to pharmaceutical compositions for the treatment or prophylaxis of the said diseases and/or disorders, which pharmaceutical compositions comprise one or more of the compounds according to the invention.

The present invention further relates to pharmaceutical compositions comprising one or more of the compounds according to this invention and a pharmaceutically acceptable carrier or diluent.

The present invention further relates to combinations comprising one or more of the compounds according to this invention and pharmaceutically acceptable auxiliaries, excipients or vehicles, e.g. for use in the treatment of those conditions mentioned above.

The present invention further relates to the use of the compounds according to this invention for the production of pharmaceutical compositions which can be used use in therapy of disorders responsive to inhibiting of PDE, such as e.g. PDE10.

The present invention further relates to compounds according to this invention having PDE, particularly PDE10, inhibiting properties.

The present invention further relates to pharmaceutical combinations or compositions according to this invention having PDE10 inhibiting properties.

The invention further relates to the use of a pharmaceutical composition comprising one or more of the compounds according to this invention as sole active ingredient(s) and a pharmaceutically acceptable carrier or diluent in the manufacture of pharmaceutical products for therapy, amelioration or prophylaxis of the illnesses, diseases, disorders or conditions mentioned above.

In addition, the present invention further relates to a use of the claimed compounds in a composition for regulating fertility in a mammal, including human, comprising administering one or more compounds according to this invention to said mammal in need thereof.

In further addition, the present invention further relates to the use of the compounds according to this invention for inhibiting spermatogenesis and/or inhibiting sperm motility in a mammal, including human.

In yet further addition, the present invention further relates to the use of the claimed compounds in a composition for regulating fertility in a mammal, including human.

Also disclosed is a commercial product which consists of a customary secondary packaging means, a primary packaging means (for example an ampoule or a blister pack) which contains a pharmaceutical composition, and, if desired, a patient information leaflet, with the pharmaceutical composition exhibiting an antagonistic effect toward type 10 cyclic nucleotide phosphodiesterases (PDE10) and leading to the attenuation of the symptoms of diseases and/or disorders which are associated with type 10 cyclic nucleotide phosphodiesterases, and with reference being made, on the secondary packaging means and/or on the patient information leaflet of the commercial product, to the suitability of the pharmaceutical composition for use in the prophylaxis or treatment of diseases and/or disorders which are associated with type 10 cyclic nucleotide phosphodiesterases, and with the pharmaceutical composition comprising one or more compounds according to this invention. The secondary packaging means, the primary packaging means containing the pharmaceutical composition and the patient information leaflet otherwise correspond to what the skilled person would regard as being the standard for drugs of this nature.

The pharmaceutical compositions according to this invention are produced using methods with which the skilled person is familiar. When employed in pharmaceutical compositions, the compounds according to the invention (= active compounds) are either used as such or, preferably, in combination with suitable pharmaceutical auxiliaries or formulating agents, for example in the form of tablets, coated (e.g. sugar-coated) tablets, capsules, caplets, suppositories, patches (e.g. as TTS), plasters, emulsions, suspensions, gels or solutions, with the content of active compound advantageously being between 0.1 and 95%, and where, by the appropriate choice of the auxiliaries, a pharmaceutical administration form (e.g. a delayed release form or an enteric form) exactly suited to the active compound and/or to the desired onset of action can be achieved.

The person skilled in the art is familiar, on the basis of his/her knowledge, with auxiliaries, vehicles, formulating agents, carriers, diluents, adjuvants or excipients which are suitable to be used for the desired pharmaceutical formulations, preparations or compositions. Beside solvents, gel-forming agents, suppository bases, tablet auxiliaries and other active carriers, it is possible to use, for example, antioxidants, dispersants, emulsifiers, antifoams, flavor corrigents, preservatives, solubilizers, colorants or, in particular, permeation promoters and complexing agents (e.g. cyclodextrines).

The administration of the pharmaceutical compositions according to the invention may be performed in any of the generally accepted modes of administration available in the art. Illustrative examples of suitable modes of administration include intravenous, inhalative, oral, nasal, parenteral, topical, transdermal and rectal delivery. Oral or intravenous delivery are preferred.

The pharmaceutical compositions according to the invention are prepared by processes known per se. For producing the drugs, the compounds according to the invention (= active compounds) are preferably mixed with suitable pharmaceutical auxiliary substances and further processed into suitable medicinal formulations. Suitable medicinal formulations which may be mentioned by way of example are powders, emulsions, suspensions, sprays, oils, ointments, greasy ointments, creams, pastes, gels and solutions.

The required dosage of the active compounds according to this invention can vary depending on the mode of administration, the particular condition to be treated and the effect desired. In general, satisfactory results are indicated to be obtained systemically at daily dosages of from about 0.01 to about 100 mg/kg body weight, conveniently administered, for example, in divided doses up to four times a day or in retard form.
The optimal dose and manner of administration of the active compounds necessary in each case can easily be determined by any person skilled in the art on the basis of his/her expert knowledge.

Depending upon the particular disease, to be treated or prevented, additional therapeutic active agents, which are normally administered to treat or prevent that disease, may optionally be coadministered separately, simultaneously, sequentially or chronologically staggered with the compounds according to this invention. As used herein, additional therapeutic agents that are normally administered to treat or prevent a particular disease are known as appropriate for the disease being treated.

The person skilled in the art is aware on the base of his/her expert knowledge of the total daily dosage(s) of the additional therapeutic agent(s) coadministered. Said total daily dosage(s) can vary within a wide range.

### Biological investigations

Methods to determine the activity and selectivity of a phosphodiesterase inhibitor are known to the person skilled in the art. In this connection it may be mentioned, for example, the methods described by Thompson et al. (Adv CycI NucI Res 10: 69-92, 1979), Giembycz et al. (Br J Pharmacol 118: 1945-1958, 1996) and the phosphodiesterase scintillation proximity assay of Amersham Pharmacia Biotech.

### Inhibiting the activity of PDE10A

The PDE10A is cloned into pCR2.1-Topo (Invitrogen) via PCR from human whole brain cDNA using primers OZ 353 (5'- ACCATGTTGACAGATGAAAAAGTGAAGGC -3') and OZ 317 (5'-TCAATCTTCAGATGCAGCTGCC -3'). The ORF encoding for the PDE10A is cut with EcoRV and BamHI and subcloned into SmaI and Bgl II of the expression vector pBP9 (Clontech). The encoded protein represents the PDE10A1 (GenBank Acc.-# AB020593) truncated at its N-terminus at aa 14.

The recombinant baculoviruses are prepared by means of homologous recombination in Sf9 insect cells. The expression plasmids are cotransfected with Bac-N-Blue (Invitrogen) or Baculo-Gold DNA (Pharmingen) using a standard protocol (Pharmingen). Wildtype virus-free recombinant virus supernatants are selected using plaque assay methods. After that, high-titre virus supernatants are prepared by amplifying 3 times. PDE10A1 is expressed in Sf21 cells by infecting 2x10⁶ cells/ml with an MOI (multiplicity of infection) between 1 and 10 in serum-free SF900 medium (Life Technologies, Paisley, UK). Cells are cultured at 28°C, typically for 48 hours, after which they are pelleted for 5-10 min at 1000 g and 4°C. In spinner flasks, cells are cultured at a rotational speed of 75 rpm. The SF21 insect cells are resuspended, at a concentration of approx. 1x10⁷ cells/ml, in ice-cold (4°C) homogenization buffer (20 mM Tris, pH 8.2, containing the following additions: 140 mM NaCl, 3.8 mM KCl, 1 mM EGTA, 1 mM MgCl₂, 10 mM β-mercaptoethanol, 2 mM benzamidine, 0.4 mM Pefabloc, 10 µM leupeptin, 10 µM pepstatin A, 5 µM trypsin inhibitor) and disrupted by ultrasonication on ice. The homogenate is then centrifuged for 10 min at 1000 g (4 °C) and the supernatant is stored at - 80 °C until subsequent use (see below). The protein content is determined by the Bradford method (BioRad, Munich) using BSA as the standard.

The PDE10A activity is inhibited by said compounds in a modified SPA (scintillation proximity assay) test, supplied by Amersham Pharmacia Biotech (see procedural instructions "Phosphodiesterase [3H]cAMP SPA enzyme assay, code TRKQ 7090"), carried out in 96-well microtitre plates (MTPs). The test volume was 100 µl and contained 20 mM Tris buffer (pH 7.4), 0.1 mg of BSA (bovine serum albumin)/ml, 5 mM Mg²⁺, 0.5 µM cAMP (including about 50,000 cpm of [3H]cAMP), 1 µl of the respective substance dilution in DMSO and sufficient recombinant PDE10A1 (1000xg supernatant, see above) to ensure that 15-20% of cAMP was converted under said experimental conditions. After a preincubation of 5 min at 37°C, the reaction is started by adding a substrate (cAMP) and the assays are incubated for a further 15 min; after that, they are stopped by adding SPA beads (50 µl). In accordance with the manufacturer's instructions, the SPA beads have previously been resuspended in water and diluted 1:3 (v/v) and added to IBMX (3 mM). After the beads have been sedimented (> 30 min), the MTPs are analyzed in commercially available measuring appliances and the corresponding IC₅₀ values of the compounds for the inhibition of PDE10A activity are determined from concentration-effect curves by means of non-linear regression.

Representative inhibitory values [inhibitory concentration as -IogIC₅₀ (mol/l)] which are determined for the compounds according to the invention are shown in the following table 1, in which the numbers of the compounds correspond to the numbers of the examples.
Particular interesting compounds according to this invention are those compounds mentioned in table. 1 below.

**Table 1: Inhibition of PDE10A activity**

| Compounds | -log IC₅₀ |
|---|---|
| 5, 6, 8, 9, 11, 12, 13, 18, 20, 21, 23, 24, 26 to 35, 37 to 54, 56 to 62, 64 to 67 | The inhibitory values of the mentioned Examples lie in the range from 7.01 to 9.58 |

## Claims

1. Compounds which inhibit PDE10, or salts thereof, comprising a structure element of formula X 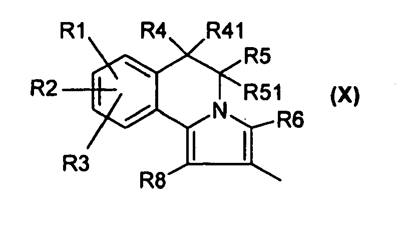 in which
R1 is halogen, nitro, 1-4C-alkyl, 1-4C-alkoxy, 1-4C-alkoxy-2-4C-alkoxy, 3-7C-cycloalkoxy or 3-7C-cycloalkylmethoxy,
R2 is 1-4C-alkoxy,
R3 is hydrogen or 1-4C-alkoxy,
R4 is hydrogen,
R41 is hydrogen,
R5 is hydrogen or 1-4C-alkyl, and
R51 is hydrogen or 1-4C-alkyl,
or
R4 is hydrogen,
R41 is hydrogen,
R5 is 1-4C-alkoxycarbonyl, and
R51 is hydrogen,
or
R4 is hydrogen,
R41 is hydrogen,
R5 is cyano, and
R51 is hydrogen,
or
R4 and R5 together form a 3-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl, and
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R8 is cyano, or -C(O)-OR9, in which
R9 is 1-4C-alkyl;
under the proviso, that,
when R8 is -C(O)-OR9, in which
R9 is 1-4C-alkyl,
then R5 is other than hydrogen;
as integral part of the overall structure of said compounds.

2. Compounds or salts thereof according to claim 1,
in which
R1 is halogen, nitro, 1-4C-alkyl, 1-4C-alkoxy, 1-4C-alkoxy-2-4C-alkoxy, 3-7C-cycloalkoxy or 3-7C-cycloalkylmethoxy,
R2 is 1-4C-alkoxy,
R3 is hydrogen or 1-4C-alkoxy,
R4 is hydrogen,
R41 is hydrogen,
R5 is hydrogen or 1-4C-alkyl, and
R51 is hydrogen or 1-4C-alkyl,
or
R4 is hydrogen,
R41 is hydrogen,
R5 is 1-4C-alkoxycarbonyl, and
R51 is hydrogen,
or
R4 and R5 together form a 3-4C-alkylene bridge and R41 and R51 are both hydrogen,
R6 is 1-6C-alkyl, amino, formyl, or 1-4C-alkyl substituted by R61, in which
R61 is 1-4C-alkoxycarbonyl, carboxyl, 1-4C-alkoxy, hydroxyl, halogen or -N(R611)R612, in which
R611 is hydrogen, 1-4C-alkyl, 3-7C-cycloalkyl or 3-7C-cycloalkyl-1-4C-alkyl, and
R612 is hydrogen or 1-4C-alkyl, or
R611 and R612 together and with inclusion of the nitrogen atom to which they are bound form a radical Het1, in which
Het1 is a 5- to 7-membered saturated heterocyclic ring radical comprising one nitrogen atom, to which
R611 and R612 are bound, and, optionally, one further heteroatom selected from a group consisting of nitrogen, oxygen and sulfur, and optionally substituted by R613 on a ring nitrogen atom, in which
R613 is 1-4C-alkyl, 3-7C-cycloalkyl, 3-7C-cycloalkyl-1-4C-alkyl, hydroxy-2-4C-alkyl, 1-4C-alkoxy-2-4C-alkyl, amino-2-4C-alkyl, mono- or di-1-4C-alkylamino-2-4C-alkyl, formyl, pyridyl or pyrimidinyl,
R8 is cyano, or -C(O)-OR9, in which
R9 is 1-4C-alkyl;
under the proviso, that,
when R8 is -C(O)-OR9, in which
R9 is 1-4C-alkyl,
then R5 is other than hydrogen;
as integral part of the overall structure of compounds which inhibit PDE10.

3. Compounds or salts thereof according to claim 1, wherein said structure element is of formula Xa or Xb 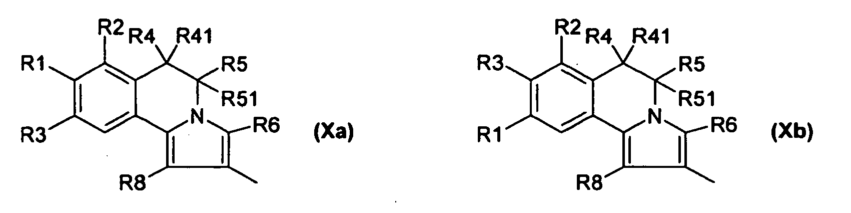 in which
as a first alternative,
R1 is chlorine or fluorine,
R2 is hydrogen,
R3 is methoxy or ethoxy,
or, as a second alternative,
R1 is methoxy or ethoxy,
R2 is hydrogen,
R3 is methoxy or ethoxy,
or, as a third alternative,
R1 is chlorine or fluorine,
R2 is methoxy or ethoxy,
R3 is methoxy or ethoxy,
or, as a fourth alternative,
R1 is methoxy or ethoxy,
R2 is methoxy or ethoxy,
R3 is methoxy or ethoxy,
R4 is hydrogen,
R41 is hydrogen,
R5 is methyl,
R51 is hydrogen,
R6 is methyl, ethyl or methoxycarbonylethyl,
R8 is cyano.

4. Compounds or salts thereof according to claim 1, wherein said structure element is of formula Xa as defined in claim 3,
in which
R1 is methoxy,
R2 is hydrogen,
R3 is methoxy,
R4 is hydrogen,
R41 is hydrogen,
R5 is methyl,
R51 is hydrogen,
R6 is methyl or methoxycarbonylethyl,
R8 is cyano.

5. Compounds or salts thereof according to claim 1, wherein said structure element is of formula Xa or Xb as defined in claim 3,
in which
R1 is 1-2C-alkoxy,
R2 is hydrogen,
R3 is 1-2C-alkoxy,
R4 is hydrogen,
R41 is hydrogen,
R5 is hydrogen, 1-2C-alkyl or cyano,
R51 is hydrogen,
R6 is 1-2C-alkyl, or 1-2C-alkyl substituted by 1-2C-alkoxycarbonyl,
R8 is cyano.

6. Compounds or salts thereof according to claim 1, wherein said structure element is of formula Xa or Xb as defined in claim 3,
in which
R1 is 1-2C-alkoxy,
R2 is hydrogen,
R3 is 1-2C-alkoxy,
R4 is hydrogen,
R41 is hydrogen,
R5 is 1-2C-alkyl or cyano,
R51 is hydrogen,
R6 is 1-2C-alkyl, or 1-2C-alkyl substituted by 1-2C-alkoxycarbonyl,
R8 is -C(O)-OR9, in which
R9 is 1-2C-alkyl,
as integral part of the overall structure of compounds which inhibit PDE10.

7. Compounds or salts thereof according to claim 1, wherein said structure element is selected from the group consisting of those structure elements of formula Xa as defined in claim 3,
in which
R1 is methoxy,
R3 is methoxy,
R4 is hydrogen,
R41 is hydrogen, and
R51 is hydrogen,
and in which the following combinations 1.) to 14.) of the substituent meanings for R2, R5, R6 and R8 apply:
| | R2 | R5 | R6 | R8 |
|---|---|---|---|---|
| 1.) | hydrogen | methyl | methyl | cyano |
| 2.) | hydrogen | methyl | methyl | ethoxycarbonyl |
| 3.) | hydrogen | methyl | 2-methoxycarbonylethyl | cyano |
| 4.) | hydrogen | methyl | 2-methoxycarbonylethyl | ethoxycarbonyl |
| 5.) | hydrogen | hydrogen | methyl | cyano |
| 6.) | hydrogen | hydrogen | 2-methoxycarbonylethyl | cyano |
| 7.) | hydrogen | cyano | methyl | cyano |
| 8.) | hydrogen | cyano | methyl | ethoxycarbonyl |
| 9.) | hydrogen | cyano | 2-methoxycarbonylethyl | cyano |
| 10.) | hydrogen | cyano | 2-methoxycarbonylethyl | ethoxycarbonyl |
| 11.) | hydrogen | methyl | methyl | methoxycarbonyl |
| 12.) | hydrogen | methyl | 2-methoxycarbonylethyl | methoxycarbonyl |
| 13.) | hydrogen | cyano | methyl | methoxycarbonyl |
| 14.) | hydrogen | cyano | 2-methoxycarbonylethyl | methoxycarbonyl |

8. Compounds or salts thereof according to claim 1, wherein said structure element is selected from the group consisting of those structure elements of formula Xa as defined in claim 3,
in which
R1 is methoxy,
R3 is methoxy,
R4 is hydrogen,
R41 is hydrogen, and
R51 is hydrogen,
and in which the following combination of the substituent meanings for R2, R5, R6 and R8 apply:
| | R2 | R5 | R6 | R8 |
|---|---|---|---|---|
| | hydrogen | methyl | methyl | cyano |

9. Compounds or salts thereof according to claim 1, wherein said structure element is of formula Xa or Xb as defined in claim 3,
in which
R1 is methoxy,
R2 is hydrogen,
R3 is methoxy,
R4 is hydrogen,
R41 is hydrogen,
R5 is methyl or cyano,
R51 is hydrogen,
R6 is methyl, or 2-methoxycarbonylethyl,
R8 is cyano.

10. Compounds or salts thereof according to any of the preceding claims, wherein the compounds are used in the manufacture of pharmaceutical compositions, such as, for example, in the manufacture of pharmaceutical compositions for the treatment of neurologic and psychiatric disorders, such as e.g. anxiety or psychotic disorders, movement disorders, obsessive/compulsive disorders, drug addictions, cognition deficiency disorders, mood disorders or mood episodes, or neurodegenerative disorders; or for the treatment of diabetes; or for the regulation of fertility of men.

11. Compounds or salts thereof according to any of the preceding claims, wherein the compounds are used in the manufacture of pharmaceutical compositions for the treatment of anxiety or psychotic disorders, such as e.g. schizophrenia, movement disorders, obsessive/compulsive disorders, drug addictions, cognition deficiency disorders, mood disorders or mood episodes, or neurodegenerative disorders.

12. A process to provide compounds according to claim 1, comprising the following steps:
a.) designing intellectually a structure of a compound comprising - as part of its overall structure - a structure element as defined in any of the claims 1 to 9;
b.) synthesizing materially a compound having the structure designed in step a.), the synthesizing comprising the steps:
(i) in a first alternative, reacting a compound of formula VIII 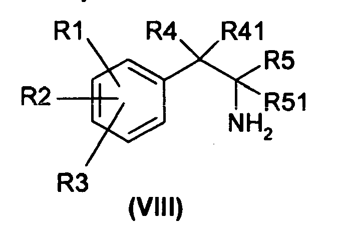 with a compound of formula VII 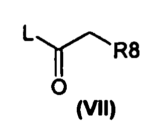 to yield a compound of formula VI 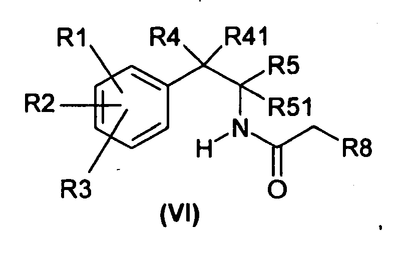 wherein L is a leaving group,
or, in a second alternative,
reacting a compound of formula VIII with a compound of formula VII 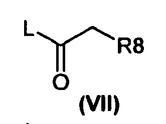 by reaction with amide bond linking agents
to yield a compound of formula VI, wherein L is hydroxyl,
(ii) subjecting the resulting compound of formula VI to a cyclocondensation to yield a compound of formula IV 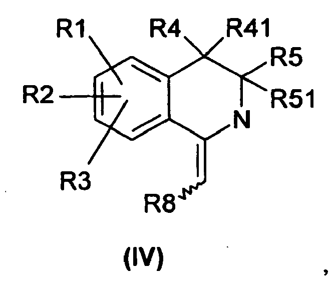 and
(iii) converting the compound of formula IV either with an aldehyde and a compound of formula III 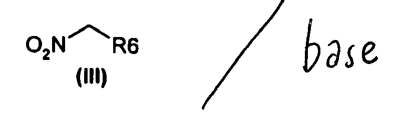 or with a nitroalkene into a compound of formula X 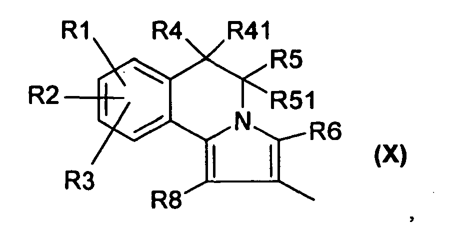 in which R1, R2, R3, R4, R41, R5, R51, R6 and R8 of formulae X, VIII, VII, VI, IV and III have the meanings as defined in claims 1-9.

13. A process for providing PDE10 inhibitors of the pyrrolodihydroisoquinoline class comprising the following steps:
a.) selecting intellectually a structure of a compound of the pyrrolodihydroisoquinoline class;
b.) modifying intellectually said selected structure in such a way that the modified structure comprises - as part of its overall structure - a structure element as defined in any of the claims 1 to 9;
c.) synthesizing materially a compound having said modified structure, the synthesizing being according to the synthesis scheme as claimed in claim 12.

14. A process to provide compounds according to claim 1, comprising the following steps:
(i) in a first alternative, reacting a compound of formula VIII 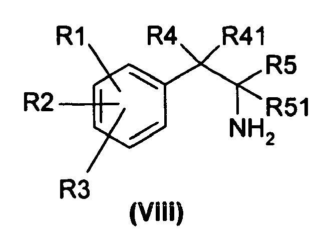 with a compound of formula VII 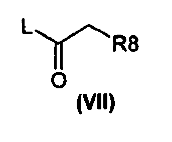 to yield a compound of formula VI 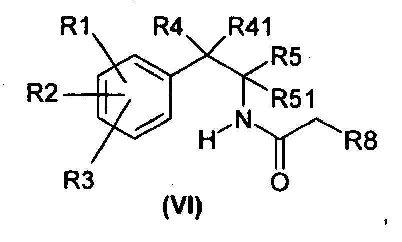 wherein L is a leaving group,
or, in a second alternative,
reacting a compound of formula VIII with a compound of formula VII 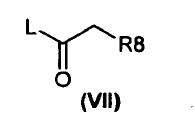 by reaction with amide bond linking agents
to yield a compound of formula VI, wherein L is hydroxyl,
(ii) subjecting the resulting compound of formula VI to a cyclocondensation to yield a compound of formula IV 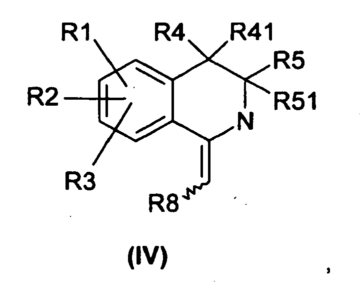 and
(iii)converting the compound of formula IV either with an aldehyde and a compound of formula III 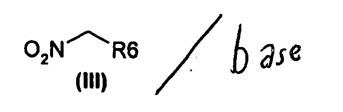 or with a nitroalkene into a compound of formula X 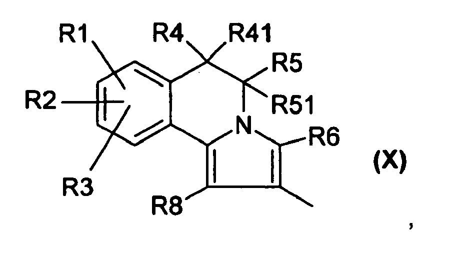
in which R1, R2, R3, R4, R41, R5, R51, R6 and R8 of formulae X, VIII, VII, VI, IV and III have the meanings as defined in claims 1-9.

## Patentansprüche

1. PDE10-hemmende Verbindungen und deren Salze, enthaltend ein Strukturelement der Formel X 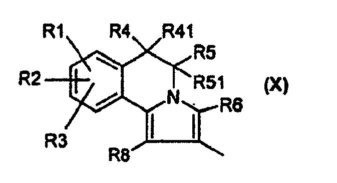 wobei
R1 für Halogen, Nitro, 1-4C-Alkyl, 1-4C-Alkoxy, 1-4C-Alkoxy-2-4C-alkoxy, 3-7C-Cycloalkoxy oder 3-7C-Cycloalkylmethoxy steht,
R2 für 1-4C-Alkoxy steht,
R3 für Wasserstoff oder 1-4C-Alkoxy steht,
R4 für Wasserstoff steht,
R41 für Wasserstoff steht,
R5 für Wasserstoff oder 1-4C-Alkyl steht, und
R51 für Wasserstoff oder 1-4C-Alkyl steht,
oder
R4 für Wasserstoff steht,
R41 für Wasserstoff steht,
R5 für 1-4C-Alkoxycarbonyl steht, und
R51 für Wasserstoff steht,
oder
R4 für Wasserstoff steht,
R41 für Wasserstoff steht,
R5 für Cyano steht, und
R51 für Wasserstoff steht,
oder
R4 und R5 zusammen eine 3-4C-Alkylenbrücke bilden und R41 und R51 beide für Wasserstoff stehen,
R6 für 1-6C-Alkyl, Amino, Formyl oder durch R61-substituiertes 1-4C-Alkyl steht, wobei
R61 für 1-4C-Alkoxycarbonyl, Carboxyl, 1-4C-Alkoxy, Hydroxy, Halogen oder -N(R611)R612 steht, wobei
R611 für Wasserstoff, 1-4C-Alkyl, 3-7C-Cycloalkyl oder 3-7C-Cycloalkyl-1-4C-alkyl steht, und
R612 für Wasserstoff oder 1-4C-Alkyl steht, oder
R611 und R612 zusammen und unter Einschluß des Stickstoffatoms, an das sie gebunden sind, einen Rest Het1 bilden, wobei
Het1 für einen 5- bis 7-gliedrigen gesättigten heterozyklischen Ringrest steht, der ein Stickstoffatom enthält, an das R611 und R612 gebunden sind, und gegebenenfalls ein weiteres Heteroatom ausgewählt aus der aus Stickstoff, Sauerstoff und Schwefel bestehenden Gruppe enthält und gegebenenfalls an einem Ringstickstoffatom durch R613 substituiert ist, wobei
R613 für 1-4C-Alkyl, 3-7C-Cycloalkyl, 3-7C-Cycloalkyl-1-4C-alkyl, Hydroxy-2-4C-alkyl, 1-4C-alkoxy-2-4C-alkyl, Amino-2-4C-alkyl, Mono-oder Di-1-4C-alkylamino-2-4C-alkyl, Formyl, Pyridyl oder Pyrimidinyl steht,
R8 für Cyano oder -C(O)-OR9 steht, wobei
R9 für 1-4C-Alkyl steht;
mit der Maßgabe, daß
wenn
R8 für -C(O)-OR9 steht, wobei
R9 für 1-4C-Alkyl steht,
R5 nicht für Wasserstoff steht;
als integrales Teil der Gesamtstruktur dieser Verbindung.

2. Verbindungen und Salze davon nach Anspruch 1,
wobei
R1 für Halogen, Nitro, 1-4C-Alkyl, 1-4C-Alkoxy, 1-4C-Alkoxy-2-4C-alkoxy, 3-7C-Cycloalkoxy oder 3-7C-Cycloalkylmethoxy steht,
R2 für 1-4C-alkoxy steht,
R3 für Wasserstoff oder 1-4C-Alkoxy steht,
R4 für Wasserstoff steht,
R41 für Wasserstoff steht,
R5 für Wasserstoff oder 1-4C-Alkyl steht, und
R51 für Wasserstoff oder 1-4C-Alkyl steht,
oder
R4 für Wasserstoff steht,
R41 für Wasserstoff steht,
R5 für 1-4C-Alkoxycarbonyl steht, und
R51 für Wasserstoff steht,
oder
R4 und R5 zusammen eine 3-4C-Alkylenbrücke bilden und R41 und R51 beide für Wasserstoff stehen,
R6 für 1-6C-Alkyl, Amino, Formyl oder durch R61-substituiertes 1-4C-Alkyl steht, wobei
R61 für 1-4C-Alkoxycarbonyl, Carboxyl, 1-4C-Alkoxy, Hydroxy, Halogen oder -N(R611)R612 steht, wobei
R611 für Wasserstoff, 1-4C-Alkyl, 3-7C-Cycloalkyl oder 3-7C-Cycloalkyl-1-4C-alkyl steht, und
R612 für Wasserstoff oder 1-4C-Alkyl steht, oder
R611 und R612 zusammen und unter Einschluß des Stickstoffatoms, an das sie gebunden sind, einen Rest Het1 bilden, wobei
Het1 für einen 5- bis 7-gliedrigen gesättigten heterozyklischen Ringrest steht, der ein Stickstoffatom enthält, an das R611 und R612 gebunden sind, und gegebenenfalls ein weiteres Heteroatom ausgewählt aus der aus Stickstoff, Sauerstoff und Schwefel bestehenden Gruppe enthält und gegebenenfalls an einem Ringstickstoffatom durch R613 substituiert ist, wobei
R613 für 1-4C-Alkyl, 3-7C-Cycloalkyl, 3-7C-Cycloalkyl-1-4C-alkyl, Hydroxy-2-4C-alkyl, 1-4C-alkoxy-2-4C-alkyl, Amino-2-4C-alkyl, Mono-oder Di-1-4C-alkylamino-2-4C-alkyl, Formyl, Pyridyl oder Pyrimidinyl steht,
R8 für Cyano oder -C(O)-OR9 steht, wobei
R9 für 1-4C-Alkyl steht;
mit der Maßgabe, daß
wenn
R8 für -C(O)-OR9 steht, wobei
R9 für 1-4C-Alkyl steht,
R5 nicht für Wasserstoff steht;
als integraler Teil der Gesamtstruktur von Verbindungen, die PDE10 hemmen.

3. Verbindungen und Salze davon nach Anspruch 1,
wobei das Strukturelement die Formel Xa oder Xb hat, 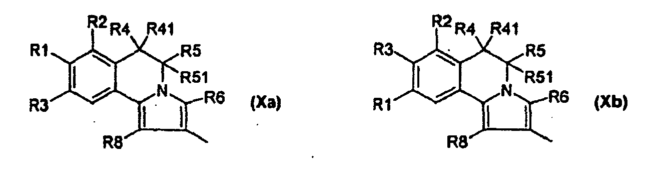 wobei,
als erste Alternative,
R1 für Chlor oder Fluor steht,
R2 für Wasserstoff steht,
R3 für Methoxy oder Ethoxy steht,
oder, als zweite Alternative,
R1 für Methoxy oder Ethoxy steht,
R2 für Wasserstoff steht,
R3 für Methoxy oder Ethoxy steht,
oder, als dritte Alternative,
R1 für Chlor oder Fluor steht,
R2 für Methoxy oder Ethoxy steht,
R3 für Methoxy oder Ethoxy steht,
oder, als vierte Alternative,
R1 für Methoxy oder Ethoxy steht,
R2 für Methoxy oder Ethoxy steht,
R3 für Methoxy oder Ethoxy steht,
R4 für Wasserstoff steht,
R41 für Wasserstoff steht,
R5 für Methyl steht,
R51 für Wasserstoff steht,
R6 für Methyl, Ethyl oder Methoxycarbonylethyl steht,
R8 für Cyano steht.

4. Verbindungen oder Salze davon nach Anspruch 1,
wobei das Strukturelement die wie in Anspruch 3 definierte Formel Xa hat,
wobei
R1 für Methoxy steht,
R2 für Wasserstoff steht,
R3 für Methoxy steht,
R4 für Wasserstoff steht,
R41 für Wasserstoff steht,
R5 für Methyl steht,
R51 für Wasserstoff steht,
R6 für Methyl oder Methoxycarbonylethyl steht,
R8 für Cyano steht.

5. Verbindungen und Salze davon nach Anspruch 1,
wobei das Strukturelement die wie in Anspruch 3 definierte Formel Xa oder Xb hat,
wobei
R1 für 1-2C-Alkoxy steht,
R2 für Wasserstoff steht,
R3 für 1-2C-Alkoxy steht,
R4 für Wasserstoff steht,
R41 für Wasserstoff steht,
R5 für Wasserstoff, 1-2C-Alkyl oder Cyano steht,
R51 für Wasserstoff steht,
R6 für 1-2C-Alkyl oder durch 1-2C-Alkoxycarbonyl-substituiertes 1-2C-Alkyl steht,
R8 für Cyano steht.

6. Verbindungen und Salze davon nach Anspruch 1,
wobei das Strukturelement die wie in Anspruch 3 definierte Formel Xa oder Xb hat,
wobei
R1 für 1-2C-Alkoxy steht,
R2 für Wasserstoff steht,
R3 für 1-2C-Alkoxy steht,
R4 für Wasserstoff steht,
R41 für Wasserstoff steht,
R5 für 1-2C-Alkyl oder Cyano steht,
R51 für Wasserstoff steht,
R6 für 1-2C-Alkyl oder durch 1-2C-Alkoxycarbonyl-substituiertes 1-2C-Alkyl steht,
R8 für -C(O)-OR9 steht, wobei
R9 für 1-2C-Alkyl steht,
als integraler Teil der Gesamtstruktur von Verbindungen, die PDE10 hemmen.

7. Verbindungen und Salze davon nach Anspruch 1,
wobei das Strukturelement ausgewählt ist aus der Gruppe bestehend aus den wie in Anspruch 3 definierten Strukturelementen der Formel Xa,
wobei
R1 für Methoxy steht,
R3 für Methoxy steht,
R4 für Wasserstoff steht,
R41 für Wasserstoff steht, und
R51 für Wasserstoff steht,
und wobei die folgenden Kombinationen 1.) bis 14.) für die Bedeutung der Substituenten R2, R5, R6 und R8 gelten:
| | R2 | R5 | R6 | R8 |
|---|---|---|---|---|
| 1.) | Wasserstoff | Methyl | Methyl | Cyano |
| 2.) | Wasserstoff | Methyl | Methyl | Ethoxycarbonyl |
| 3.) | Wasserstoff | Methyl | 2-Methoxycarbonylethyl | Cyano |
| 4.) | Wasserstoff | Methyl | 2-Methoxycarbonylethyl | Ethoxycarbonyl |
| 5.) | Wasserstoff | Wasserstoff | Methyl | Cyano |
| 6.) | Wasserstoff | Wasserstoff | 2-Methoxycarbonylethyl | Cyano |
| 7.) | Wasserstoff | Cyano | Methyl | Cyano |
| 8.) | Wasserstoff | Cyano | Methyl | Ethoxycarbonyl |
| 9.) | Wasserstoff | Cyano | 2-Methoxycarbonylethyl | Cyano |
| 10.) | Wasserstoff | Cyano | 2-Methoxycarbonylethyl | Ethoxycarbonyl |
| 11.) | Wasserstoff | Methyl | Methyl | Methoxycarbonyl |
| 12.) | Wasserstoff | Methyl | 2-Methoxycarbonylethyl | Methoxycarbonyl |
| 13.) | Wasserstoff | Cyano | Methyl | Methoxycarbonyl |
| 14.) | Wasserstoff | Cyano | 2-Methoxycarbonylethyl | Methoxycarbonyl |

8. Verbindungen und Salze davon nach Anspruch 1,
wobei das Strukturelement ausgewählt ist aus der Gruppe bestehend aus den wie in Anspruch 3 definierten Strukturelementen der Formel Xa,
wobei
R1 für Methoxy steht,
R3 für Methoxy steht,
R4 für Wasserstoff steht,
R41 für Wasserstoff steht, und
R51 für Wasserstoff steht,
und wobei die folgende Kombination für die Bedeutung der Substituenten R2, R5, R6 und R8 gilt:
| | R2 | R5 | R6 | R8 |
|---|---|---|---|---|
| | Wasserstoff | Methyl | Methyl | Cyano |

9. Verbindungen und Salze davon nach Anspruch 1,
wobei das Strukturelement die wie in Anspruch 3 definierte Formel Xa oder Xb hat,
wobei
R1 für Methoxy steht,
R2 für Wasserstoff steht,
R3 für Methoxy steht,
R4 für Wasserstoff steht,
R41 für Wasserstoff steht,
R5 für Methyl oder Cyano steht,
R51 für Wasserstoff steht,
R6 für Methyl oder 2-Methoxycarbonylethyl steht,
R8 für Cyano steht.

10. Verbindungen und Salze davon nach einem der vorhergehenden Ansprüche, wobei die Verbindungen in der Herstellung von pharmazeutischen Zusammensetzungen wie zum Beispiel bei der Herstellung von pharmazeutischen Zusammensetzungen für die Behandlung von neurologischen und psychiatrischen Störungen wie z.B. Angst oder psychotischen Störungen, Bewegungsstörungen, Zwangsneurosen, Drogen- bzw. Arzneimittelsüchten, kognitiven Funktionsstörungen, Gemütsstörungen oder Gemütsepisoden oder neurodegenerativen Störungen; oder zur Behandlung von Diabetes; oder zur Regulierung der Fruchtbarkeit des Mannes verwendet werden.

11. Verbindungen und Salze davon nach einem der vorhergehenden Ansprüche, wobei die Verbindungen bei der Herstellung von pharmazeutischen Zusammensetzungen zur Behandlung von Angst oder psychotischen Störungen wie z.B. Schizophrenie, Bewegungsstörungen, Zwangsneurosen, Drogen- bzw. Arzneimittelsüchten, kognitiven Funktionsstörungen, Gemütsstörungen oder Gemütsepisoden oder neurodegenerativen Störungen verwendet werden.

12. Verfahren zur Bereitstellung von Verbindungen nach Anspruch 1, welches die folgenden Schritte umfaßt:
a.) die intellektuelle Entwicklung einer Struktur einer Verbindung, die - als Teil ihrer Gesamtstruktur - ein wie in einem der Ansprüche 1 bis 9 definiertes Strukturelement enthält;
b.) die materielle Synthese einer Verbindung mit der in Schritt a.) entwickelten Struktur, wobei die Synthese die folgenden Schritte umfaßt:
i) in einer ersten Alternative die Umsetzung einer Verbindung der Formel VIII 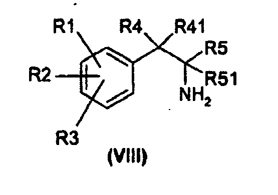 mit einer Verbindung der Formel VII 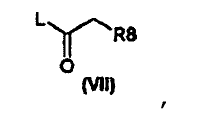 wodurch man eine Verbindung der Formel VI 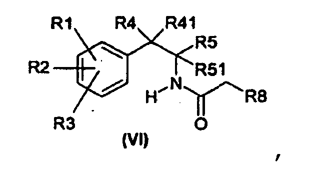 erhält, in welcher L für eine Abgangsgruppe steht,
oder, in einer zweiten Alternative,
die Umsetzung einer Verbindung der Formel VIII mit einer Verbindung der Formel VII 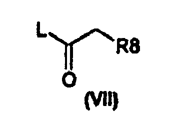 durch Umsetzung mit amidbindungsbildenden Mitteln, wodurch man eine Verbindung der Formel VI erhält, in welcher L für Hydroxy steht,
ii) die Cyclokondensation der erhaltenen Verbindung der Formel VI, wodurch man eine Verbindung der Formel IV 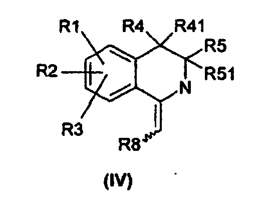 erhält, und
iii) die Umwandlung der Verbindung der Formel IV entweder mit einem Aldehyd und einer Verbindung der Formel III 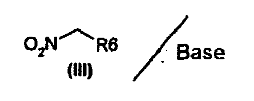 oder mit einem Nitroalken in einer Verbindung der Formel X 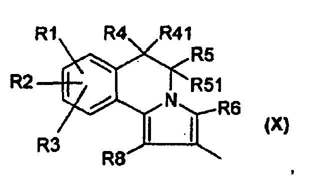
wobei R1, R2, R3, R4, R41, R5, R51, R6 und R8 in den Formeln X, VIII, VII, VI, IV und III die in den Ansprüchen 1-9 definierten Bedeutungen haben.

13. Verfahren zur Bereitstellung von PDE10-Inhibitoren der Pyrrolodihydroisochinolinklasse, welches die folgenden Schritte umfaßt:
a.) die intellektuelle Auswahl einer Struktur einer Verbindung der Pyrrolodihydroisochinolinklasse;
b.) die intellektuelle Modifikation dieser ausgewählten Struktur in einer Weise, so daß die modifizierte Struktur - als Teil ihrer Gesamtstruktur - ein wie in einem der Ansprüche 1 bis 9 definiertes Strukturelement enthält;
c.) die materielle Synthese einer Verbindung mit dieser modifizierten Struktur, wobei die Synthese gemäß dem in Anspruch 12 beanspruchten Syntheseschema erfolgt.

14. Verfahren zur Bereitstellung von Verbindungen nach Anspruch 1, welches die folgenden Schritte umfaßt:
(i) in einer ersten Alternative die Umsetzung einer Verbindung der Formel VIII 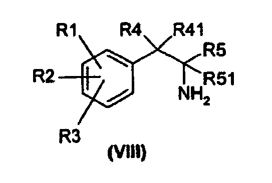 mit einer Verbindung der Formel VII 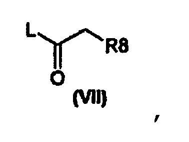 wodurch man eine Verbindung der Formel VI 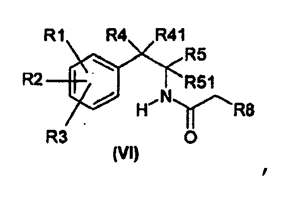 in welcher L für eine Abgangsgruppe steht, erhält oder, in einer zweiten Alternative, die Umsetzung einer Verbindung der Formel VIII mit einer Verbindung der Formel VII 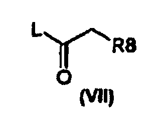 durch Umsetzung mit amidbindungsbildenden Mitteln, wodurch man eine Verbindung der Formel VI erhält, in welcher L für Hydroxy steht,
(ii) die Cyclokondensation der erhaltenen Verbindung der Formel VI, wodurch man eine Verbindung der Formel IV erhält 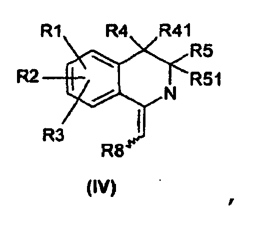 und
(iii)die Umwandlung der Verbindung der Formel IV entweder mit einem Aldehyd und einer Verbindung der Formel III 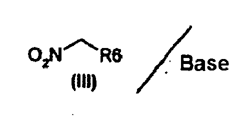 oder mit einem Nitroalken in eine Verbindung der Formel X 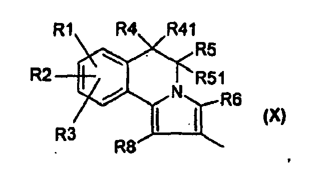 wobei R1, R2, R3, R4, R41, R5, R51, R6 und R8 in den Formeln X, VIII, VII, VI, IV und III die in den Ansprüchen 1-9 definierten Bedeutungen haben.

## Revendications

1. Composés qui inhibent la PDE10, ou leurs sels, comprenant un élément structural de formule X 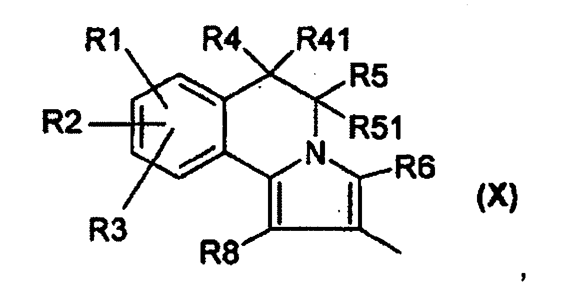 dans laquelle
R1 est un atome d'halogène, un groupe nitro, un groupe alkyle en C₁-C₄, un groupe alcoxy en C₁-C₄, un groupe C₁-C₄-alcoxy-C₂-C₄-alcoxy, un groupe cycloalcoxy en C₃-C₇ ou un groupe C₃-C₇-cycloalkylméthoxy,
R2 est un groupe alcoxy en C₁-C₄,
R3 est un atome d'hydrogène ou un groupe alcoxy en C₁-C₄,
R4 est un atome d'hydrogène,
R41 est un atome d'hydrogène,
R5 est un atome d'hydrogène ou un groupe alkyle en C₁-C₄, et
R51 est un atome d'hydrogène ou un groupe alkyle en C₁-C₄,
ou
R4 est un atome d'hydrogène,
R41 est un atome d'hydrogène,
R5 est un groupe C₁-C₄-alcoxycarbonyle, et
R51 est un atome d'hydrogène,
ou
R4 est un atome d'hydrogène,
R41 est un atome d'hydrogène,
R5 est un groupe cyano, et
R51 est un atome d'hydrogène,
ou
R4 et R5 forment conjointement un pont alkylène en C₃-C₄ et R41 et R51 sont tous les deux un atome d'hydrogène,
R6 est un groupe alkyle en C₁-C₆, un groupe amino, un groupe formyle, ou un groupe alkyle en C₁-C₄ substitué par R61, où
R61 est un groupe C₁-C₄-alcoxycarbonyle, un groupe carboxyle, un groupe alcoxy en C₁-C₄, un groupe hydroxyle, un atome d'halogène ou un groupe -N(R611)R612, dans lequel
R611 est un atome d'hydrogène, un groupe alkyle en C₁-C₄, un groupe cycloalkyle en C₃-C₇ ou un groupe C₃-C₇-cycloalkyl-C₁-C₄-alkyle, et
R612 est un atome d'hydrogène ou un groupe alkyle en C₁-C₄, ou
R611 et R612, conjointement et en incluant l'atome d'azote auquel ils sont liés, forment un radical Hetl, où
Het1 est un radical de noyau hétérocyclique saturé à 5-7 chaînons, renfermant un atome d'azote, auquel R611 et R612 sont liés, et éventuellement un hétéroatome supplémentaire choisi parmi un groupe constitué d'un atome d'azote, d'un atome d'oxygène et d'un atome de soufre, et éventuellement substitué par R613 sur un atome d'azote du noyau, où
R613 est un groupe alkyle en C₁-C₄, un groupe cycloalkyle en C₃-C₇, un groupe C₃-C₇-cycloalkyl-C₁-C₄-alkyle, un groupe hydroxy-C₂-C₄-alkyle, un groupe C₁-C₄-alcoxy-C₂-C₄-alkyle, un groupe amino-C₂-C₄-alkyle, un groupe mono-ou di-C₁-C₄-alkylamino-C₂-C₄-alkyle, un groupe formyle, un groupe pyridyle ou un groupe pyrimidinyle,
R8 est un groupe cyano ou un groupe -C(O)-OR9,
dans lequel
R9 est un groupe alkyle en C₁-C₄ ;
à condition que,
lorsque
R8 est un groupe -C(O)-OR9, dans lequel
R9 est un groupe alkyle en C₁-C₄,
alors
R5 est autre qu'un atome d'hydrogène ; faisant partie intégrante de la structure globale desdits composés.

2. Composés ou leurs sels selon la revendication 1,
dans lesquels
R1 est un atome d'halogène, un groupe nitro, un groupe alkyle en C₁-C₄, un groupe alcoxy en C₁-C₄, un groupe C₁-C₄-alcoxy-C₂-C₄-alcoxy, un groupe cycloalcoxy en C₃-C₇ ou un groupe C₃-C₇-cycloalkylméthoxy,
R2 est un groupe alcoxy en C₁-C₄,
R3 est un atome d'hydrogène ou un groupe alcoxy en C₁-C₄,
R4 est un atome d'hydrogène,
R41 est un atome d'hydrogène,
R5 est un atome d'hydrogène ou un groupe alkyle en C₁-C₄, et
R51 est un atome d'hydrogène ou un groupe alkyle en C₁-C₄,
ou
R4 est un atome d'hydrogène,
R41 est un atome d'hydrogène,
R5 est un groupe C₁-C₄-alcoxycarbonyle, et
R51 est un atome d'hydrogène,
ou
R4 et R5 forment conjointement un pont alkylène en C₃-C₄ et R41 et R51 sont tous les deux un atome d'hydrogène,
R6 est un groupe alkyle en C₁-C₆, un groupe amino, un groupe formyle, ou un groupe alkyle en C₁-C₄ substitué par R61, où
R61 est un groupe C₁-C₄-alcoxycarbonyle, un groupe carboxyle, un groupe alcoxy en C₁-C₄, un groupe hydroxyle, un atome d'halogène ou un groupe -N(R611)R612, dans lequel
R611 est un atome d'hydrogène, un groupe alkyle en C₁-C₄, un groupe cycloalkyle en C₃-C₇ ou un groupe C₃-C₇-cycloalkyl-C₁-C₄-alkyle, et
R612 est un atome d'hydrogène ou un groupe alkyle en C₁-C₄, ou
R611 et R612, conjointement et en incluant l'atome d'azote auquel ils sont liés, forment un radical Het1, où
Het1 est un radical de noyau hétérocyclique saturé à 5-7 chaînons, renfermant un atome d'azote, auquel R611 et R612 sont liés, et éventuellement un hétéroatome supplémentaire choisi parmi un groupe constitué d'un atome d'azote, d'un atome d'oxygène et d'un atome de soufre, et éventuellement substitué par R613 sur un atome d'azote du noyau, où
R613 est un groupe alkyle en C₁-C₄, un groupe cycloalkyle en C₃-C₇, un groupe C₃-C₇-cycloalkyl-C₁-C₄-alkyle, un groupe hydroxy-C₂-C₄-alkyle, un groupe C₁-C₄-alcoxy-C₂-C₄-alkyle, un groupe amino-C₂-C₄-alkyle, un groupe mono-ou di-C₁-C₄-alkylamino-C₂-C₄-alkyle, un groupe formyle, un groupe pyridyle ou un groupe pyrimidinyle,
R8 est un groupe cyano ou un groupe -C(O)-OR9,
dans lequel
R9 est un groupe alkyle en C₁-C₄ ;
à condition que,
lorsque
R8 est un groupe -C(O)-OR9, dans lequel
R9 est un groupe alkyle en C₁-C₄,
alors
R5 est autre qu'un atome d'hydrogène ;
faisant partie intégrante de la structure globale de composés qui inhibent la PDE10.

3. Composés ou leurs sels selon la revendication 1, dans lesquels ledit élément structural est de formule Xa ou Xb 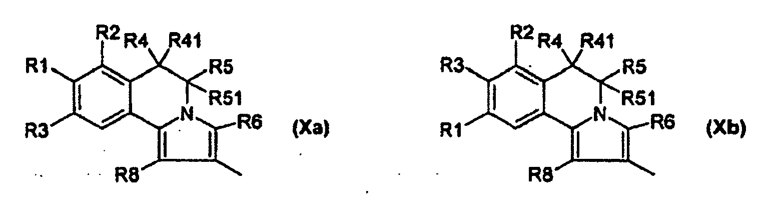 dans lesquelles
en tant que première variante,
R1 est un atome de chlore ou un atome de fluor,
R2 est un atome d'hydrogène,
R3 est un groupe méthoxy ou un groupe éthoxy,
ou, en tant que deuxième variante,
R1 est un groupe méthoxy ou un groupe éthoxy,
R2 est un atome d'hydrogène,
R3 est un groupe méthoxy ou un groupe éthoxy,
ou, en tant que troisième variante,
R1 est un atome de chlore ou un atome de fluor,
R2 est un groupe méthoxy ou un groupe éthoxy,
R3 est un groupe méthoxy ou un groupe éthoxy,
ou, en tant que quatrième variante,
R1 est un groupe méthoxy ou un groupe éthoxy,
R2 est un groupe méthoxy ou un groupe éthoxy,
R3 est un groupe méthoxy ou un groupe éthoxy,
R4 est un atome d'hydrogène,
R41 est un atome d'hydrogène,
R5 est un groupe méthyle,
R51 est un atome d'hydrogène,
R6 est un groupe méthyle, un groupe éthyle ou un groupe méthoxycarbonyléthyle,
R8 est un groupe cyano.

4. Composés ou leurs sels selon la revendication 1, dans lesquels ledit élément structural est de formule Xa telle que définie dans la revendication 3, dans laquelle
R1 est un groupe méthoxy,
R2 est un atome d'hydrogène,
R3 est un groupe méthoxy,
R4 est un atome d'hydrogène,
R41 est un atome d'hydrogène,
R5 est un groupe méthyle,
R51 est un atome d'hydrogène,
R6 est un groupe méthyle ou un groupe méthoxycarbonyléthyle,
R8 est un groupe cyano.

5. Composés ou leurs sels selon la revendication 1,
dans lesquels ledit élément structural est de formules Xa ou Xb telles que définies dans la revendication 3,
dans lesquelles
R1 est un groupe alcoxy en C₁-C₂,
R2 est un atome d'hydrogène,
R3 est un groupe alcoxy en C₁-C₂,
R4 est un atome d'hydrogène,
R41 est un atome d'hydrogène,
R5 est un atome d'hydrogène, un groupe alkyle en C₁-C₂ ou un groupe cyano,
R51 est un atome d'hydrogène,
R6 est un groupe alkyle en C₁-C₂, ou un groupe alkyle en C₁-C₂ substitué par un groupe C₁-C₂-alcoxycarbonyle,
R8 est un groupe cyano.

6. Composés ou leurs sels selon la revendication 1,
dans lesquels ledit élément structural est de formules Xa ou Xb telles que définies dans la revendication 3,
dans lesquelles
R1 est un groupe alcoxy en C₁-C₂,
R2 est un atome d'hydrogène,
R3 est un groupe alcoxy en C₁-C₂,
R4 est un atome d'hydrogène,
R41 est un atome d'hydrogène,
R5 est un groupe alkyle en C₁-C₂ ou un groupe cyano,
R51 est un atome d'hydrogène,
R6 est un groupe alkyle en C₁-C₂, ou un groupe alkyle en C₁-C₂ substitué par un groupe C₁-C₂-alcoxycarbonyle,
R8 est un groupe -C(O)-OR9, dans lequel
R9 est un groupe alkyle en C₁-C₂,
faisant partie intégrante de la structure globale de composés qui inhibent la PDE10.

7. Composés ou leurs sels selon la revendication 1,
dans lesquels ledit élément structural est choisi parmi le groupe constitué des éléments structuraux de formule Xa telle que définie dans la revendication 3,
dans laquelle
R1 est un groupe méthoxy,
R3 est un groupe méthoxy,
R4 est un atome d'hydrogène,
R41 est un atome d'hydrogène, et
R51 est un atome d'hydrogène,
et dans laquelle les combinaisons 1.) à 14.) suivantes des significations des substituants pour R2, R5, R6 et R8 s'appliquent :
| | R2 | R5 | R6 | R8 |
|---|---|---|---|---|
| 1.) | hydrogène | méthyle | méthyle | cyano |
| 2.) | hydrogène | méthyle | méthyle | éthoxycarbonyle |
| 3.) | hydrogène | méthyle | 2-méthoxycarbonyléthyle | cyano |
| 4.) | hydrogène | méthyle | 2-méthoxycarbonyléthyle | éthoxycarbonyle |
| 5.) | hydrogène | hydrogène | méthyle | cyano |
| 6.) | hydrogène | hydrogène | 2-méthoxycarbonyléthyle | cyano |
| 7.) | hydrogène | cyano | méthyle | cyano |
| 8.) | hydrogène | cyano | méthyle | éthoxycarbonyle |
| 9.) | hydrogène | cyano | 2-méthoxycarbonyléthyle | cyano |
| 10.) | hydrogène | cyano | 2-méthoxycarbonyléthyle | éthoxycarbonyle |
| 11.) | hydrogène | méthyle | méthyle | méthoxycarbonyle |
| 12.) | hydrogène | méthyle | 2-méthoxycarbonyléthyle | méthoxycarbonyle |
| 13.) | hydrogène | cyano | méthyle | méthoxycarbonyle |
| 14.) | hydrogène | cyano | 2-méthoxycarbonyléthyle | méthoxycarbonyle |

8. Composés ou leurs sels selon la revendication 1, dans lesquels ledit élément structural est choisi parmi le groupe constitué des éléments structuraux de formule Xa telle que définie dans la revendication 3,
dans laquelle
R1 est un groupe méthoxy,
R3 est un groupe méthoxy,
R4 est un atome d'hydrogène,
R41 est un atome d'hydrogène, et
R51 est un atome d'hydrogène,
et dans laquelle la combinaison suivante des significations des substituants pour R2, R5, R6 et R8 s'applique :
| | R2 | R5 | R6 | R8 |
|---|---|---|---|---|
| | hydrogène | méthyle | méthyle | cyano |

9. Composés ou leurs sels selon la revendication 1,
dans lesquels ledit élément structural est de formules Xa ou Xb telles que définies dans la revendication 3,
dans lesquelles
R1 est un groupe méthoxy,
R2 est un atome d'hydrogène,
R3 est un groupe méthoxy,
R4 est un atome d'hydrogène,
R41 est un atome d'hydrogène,
R5 est un groupe méthyle ou un groupe cyano,
R51 est un atome d'hydrogène,
R6 est un groupe méthyle ou un groupe 2-méthoxycarbonyléthyle,
R8 est un groupe cyano.

10. Composés ou leurs sels selon l'une quelconque des revendications précédentes, où les composés sont utilisés pour la fabrication de compositions pharmaceutiques, comme, par exemple, pour la fabrication de compositions pharmaceutiques destinées au traitement de troubles neurologiques et psychiatriques, tels que, par exemple, des troubles de l'anxiété ou psychotiques, des troubles du mouvement, des troubles obsessifs/compulsifs, des toxicomanies, des troubles de déficience cognitive, des troubles de l'humeur ou d'épisodes de l'humeur, ou des troubles neurodégénératifs ; ou au traitement du diabète ; ou à la régulation de la fertilité de l'homme.

11. Composés ou leurs sels selon l'une quelconque des revendications précédentes, où les composés sont utilisés pour la fabrication de compositions pharmaceutiques destinées au traitement de troubles de l'anxiété ou psychotiques, tels que, par exemple, la schizophrénie, des troubles du mouvement, des troubles obsessifs/compulsifs, des toxicomanies, des troubles de déficience cognitive, des troubles de l'humeur ou d'épisodes de l'humeur, ou des troubles neurodégénératifs.

12. Procédé pour procurer des composés selon la revendication 1, comprenant les étapes consistant à :
a.) concevoir intellectuellement une structure d'un composé comprenant - en tant que partie de sa structure globale - un élément structural tel que défini dans l'une quelconque des revendications 1 à 9 ;
b.) synthétiser matériellement un composé présentant la structure conçue dans l'étape a.), la synthèse comprenant les étapes consistant à :
(i) dans une première variante, faire réagir un composé de formule VIII 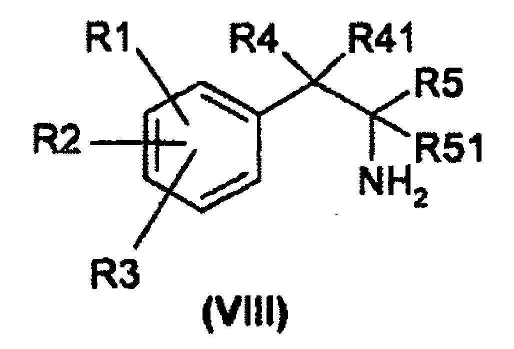 avec un composé de formule VII 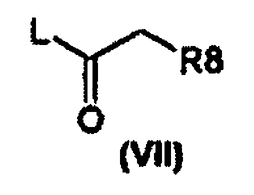 pour donner lieu à un composé de formule VI 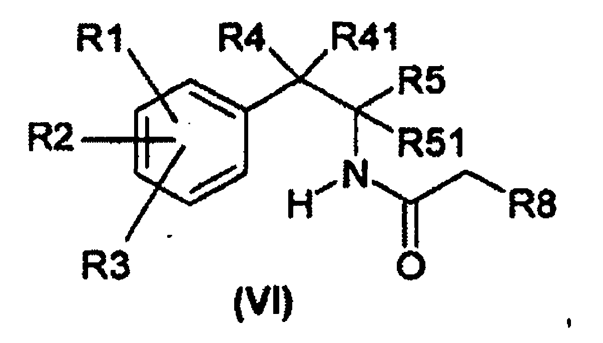 où L est un groupe partant,
ou, dans une deuxième variante,
faire réagir un composé de formule VIII avec un composé de formule VII 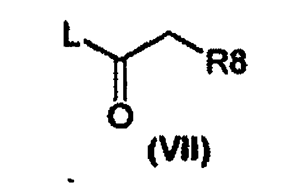 par réaction avec des agents de liaison à liaison amide,
pour donner lieu à un composé de formule VI, où L est un groupe hydroxyle,
(ii) soumettre le composé résultant de formule VI à une cyclocondensation pour donner lieu à un composé de formule IV 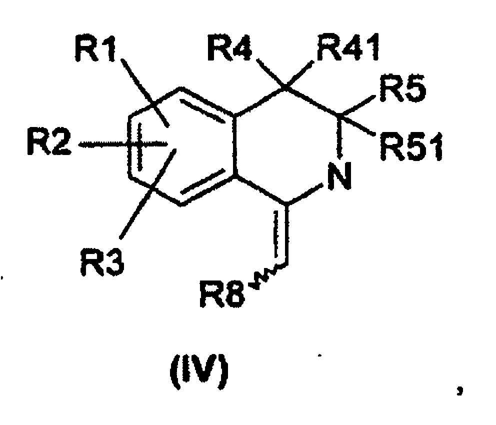 et
(iii) convertir le composé de formule IV soit avec un aldéhyde et un composé de formule III 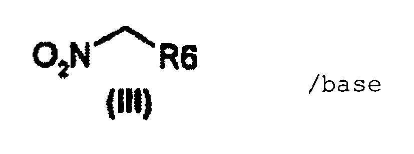 soit avec un nitroalcène, en un composé de formule X 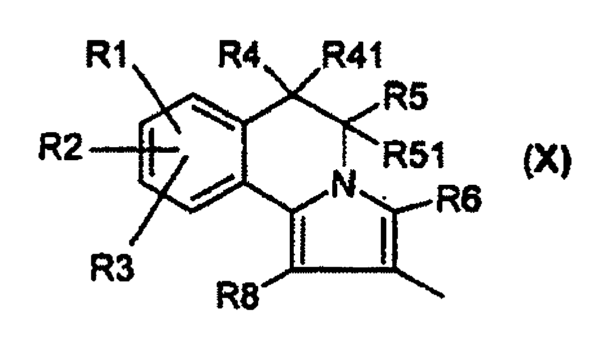
où R1, R2, R3, R4, R41, R5, R51, R6 et R8 des formules X, VIII, VII, VI, IV, et III présentent les significations telles que définies dans les revendications 1 à 9.

13. Procédé pour procurer des inhibiteurs de la PDE10 de la classe des pyrrolodihydroisoquinoléines, comprenant les étapes consistant à :
a.) choisir intellectuellement une structure d'un composé de la classe des pyrrolodihydroisoquinoléines ;
b.) modifier intellectuellement ladite structure choisie de telle sorte que la structure modifiée comprenne - en tant que partie de sa structure globale - un élément structural tel que défini dans l'une quelconque des revendications 1 à 9 ;
c.) synthétiser matériellement un composé présentant ladite structure modifiée, la synthèse étant conforme au schéma de synthèse selon la revendication 12.

14. Procédé pour procurer des composés selon la revendication 1, comprenant les étapes consistant à :
(i) dans une première variante, faire réagir un composé de formule VIII 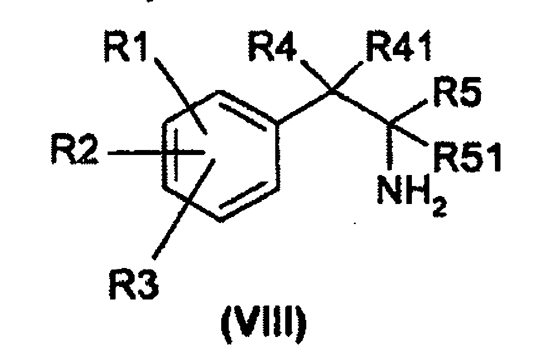 avec un composé de formule VII 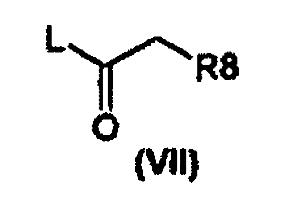 pour donner lieu à un composé de formule VI 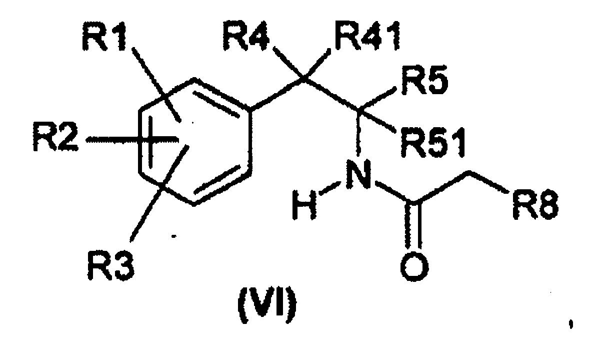 où L est un groupe partant,
ou, dans une deuxième variante,
faire réagir un composé de formule VIII avec un composé de formule VII 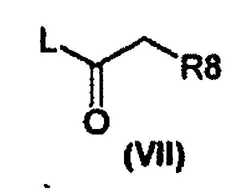 par réaction avec des agents de liaison à liaison amide,
pour donner lieu à un composé de formule VI, où L est un groupe hydroxyle,
(ii) soumettre le composé résultant de formule VI à une cyclocondensation pour donner lieu à un composé de formule IV 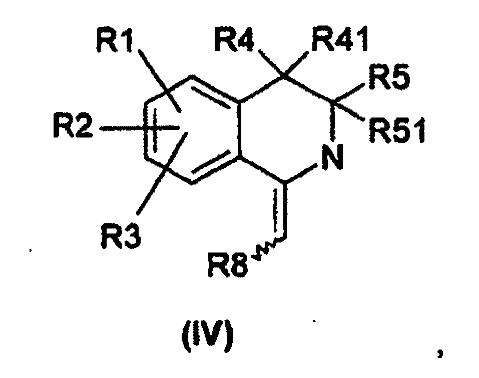 et
(iii) convertir le composé de formule IV soit avec un aldéhyde et un composé de formule III 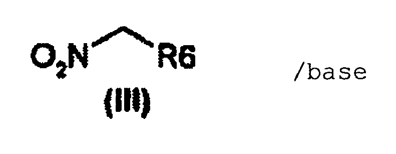 soit avec un nitroalcène, en un composé de formule X 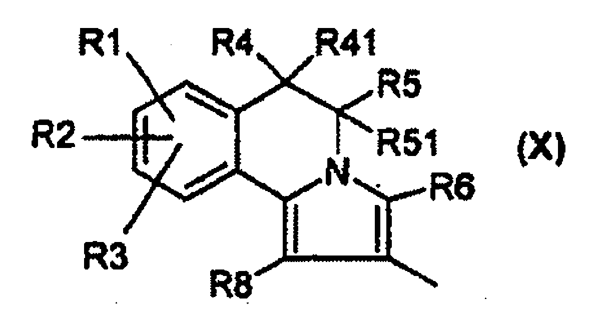
où R1, R2, R3, R4, R41, R5, R51, R6 et R8 des formules X, VIII, VII, VI, IV, et III présentent les significations telles que définies dans les revendications 1 à 9.
